(19) Europäisches Patentamt
European Patent Office
Office européen des brevets

(11) EP 2 441 771 A1

(12) **EUROPÄISCHE PATENTANMELDUNG**

(43) Veröffentlichungstag:
**18.04.2012 Patentblatt 2012/16**

(51) Int Cl.:
***C07K 14/33*** *(2006.01)* ***C12N 9/04*** *(2006.01)*

(21) Anmeldenummer: **10187411.3**

(22) Anmeldetag: **13.10.2010**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **PharmaZell GmbH**
**83064 Raubling (DE)**

(72) Erfinder:
- **Weuster-Botz, Dirk**
**80634 München (DE)**
- **Braun, Michael**
**97980 Bad-Mergentheim-Edelfingen (DE)**
- **Aigner, Arno**
**83104 Tuntenhausen (DE)**

(74) Vertreter: **Reitstötter - Kinzebach**
**Patentanwälte**
**Sternwartstrasse 4**
**81679 München (DE)**

(54) **Neue 12alpha-Hydroxysteroid- dehydrogenase-Mutanten, deren Herstellung deren Verwendung**

(57) Die vorliegende Erfindung betrifft neue 12α-Hydroxysteroiddehydrogenase-Mutanten, dafür kodierende Nukleinsäuresequenzen, Expressionskassetten und Vektoren; rekombinante Mikroorganismen, enthaltend entsprechende kodierende Nukleinsäuresequenzen; Verfahren zur Herstellung solcher 12 α-Hydroxysteroiddehydrogenase-Mutanten; Verfahren zur enzymatischen Oxidation von 12α-Hydroxysteroiden unter Verwendung derartiger Mutanten, Verfahren zur enzymatischen Reduktion von 12-Ketosteroiden unter Verwendung solcher Enzyme; Verfahren zur qualitativen oder quantitativen Bestimmung von 12-Ketosteroiden bzw. 12α-Hydroxysteroiden unter Verwendung der erfindungsgemäßen 12α-Hydroxysteroiddehydrogenase-Mutanten; sowie ein Verfahren zur Herstellung von Ursodesoxycholsäure, umfassend die enzymkatalysierte Cholsäureoxidation unter Verwendung der erfindungsgemäßen 12α-Hydroxysteroiddehydrogenase-Mutanten.

EP 2 441 771 A1

## Beschreibung

Die vorliegende Erfindung betrifft neue 12α-Hydroxysteroiddehydrogenase-Mutanten, dafür kodierende Nukleinsäuresequenzen, Expressionskassetten und Vektoren; rekombinante Mikroorganismen, enthaltend entsprechende kodierende Nukleinsäuresequenzen; Verfahren zur Herstellung solcher 12 α-Hydroxysteroiddehydrogenase-Mutanten; Verfahren zur enzymatischen Oxidation von 12α-Hydroxysteroiden unter Verwendung derartiger Mutanten, Verfahren zur enzymatischen Reduktion von 12-Ketosteroiden unter Verwendung solcher Enzyme; Verfahren zur qualitativen oder quantitativen Bestimmung von 12-Ketosteroiden bzw. 12α-Hydroxysteroiden unter Verwendung der erfindungsgemäßen 12α-Hydroxysteroiddehydrogenase-Mutanten; sowie ein Verfahren zur Herstellung von Ursodesoxycholsäure, umfassend die enzymkatalysierte Cholsäureoxidation unter Verwendung der erfindungsgemäßen 12α-Hydroxysteroiddehydrogenase-Mutanten.

## Hintergrund der Erfindung

Die 12α-Hydroxysteroiddehydrogenase (12α-HSDH) ist ein für die stereospezifische Synthese, wie z.B. die Oxidation von 12α-Hydroxysteroiden, wie die Cholsäure, wichtiger Biokatalysator. Ebenso kann die 12α-HSDH zur Reduktion von 12-Ketosteroiden, wie z.B. 12-Keto-Chenodeoxycholsäure, eingesetzt werden.

12α-HSDHs gehören der Enzymklasse EC 1.1.1.176 an und werden hauptsächlich in Bakterien der Gattung *Clostridium* gefunden. Es existieren sowohl $NADP^+$-abhängige (Harris and Hylemon (1978) Biochim Biophys Acta 528 (1): 148-57), als auch $NAD^+$-abhängige Vertreter (Macdonald et al. 1976) Biochim Biophys Acta 450(2): 142-53.

Die jeweilige Redox-Reaktion verläuft dabei unter Bildung bzw. Verbrauch des entsprechenden Cofaktors bzw. Redoxpartners, d.h. NAD/NADH bzw. NADP/NADPH.

Untersuchungen zu einer 12α-HSDH aus *Clostridium sp.* group P strain 48 - 50 und deren partielle Reinigung durch $NAD^+$ und $NADP^+$-Sepharose Chromatographie wurde von Mahony et al. (Mahony, D.E., et al Appl Environ Microbiol, 1977. 34(4): p. 419-23) bzw. Macdonald et al, (Macdonald, I.A., et al, Journal of Lipid Research, 1979, 20 234-239) beschrieben.

Ein entsprechend hergestellter 12α-HSDH-haltiger Proteinextrakt aus *Clostridium* Gruppe P wurde von Sutherland et al. im Rahmen von drei verschiedenen Synthesewegen von Ursodesoxycholsäure aus Cholsäure eingesetzt. Einer der Synthesewege umfasste dabei die enzymkatalysierte Oxidation von Cholsäure zu 12-Ketochenodesoxycholsäure (12-Keto-CDCS) (Sutherland, J.D., et al, Prep Biochem, 1982. 12(4): p. 307-21). Als Enzympräparation kam dabei Zelllysat aus *Clostridium sp.* group P strain 48 -50 DSM 4029 zum Einsatz. Die Reaktion benötigt stöchiometrische Mengen des Kofaktors $NADP^+$.

Die von einer $NAD^+$-abhängigen 12α-HSDH katalysierte Cholsäureoxidation mit Kofaktorregenerierung durch Lactatdehydrogenase (LDH; Umsetzung von Pyruvat zu Lactat unter Regenerierung von $NAD^+$) wird in der EP-A-1 731 618 beschrieben.

Braun, M., et al., Eur J Biochem, 1991. 196(2): p. 439-50 beschreiben eine zweistufige Aufreinigungsstrategie für das Wildtyp-Enzym aus *Clostridium sp.* group P strain 48-50 und eine N-terminale Aminosäuresequenz. Lediglich eine N-terminale Teil-Sequenz, umfassend 29 Aminosäurereste wird dort offenbart.

Die WO 2009/118176 beschreibt die rekombinante Herstellung von 12α-HSDH aus Clostridium sp group P strain 48-50 und offenbart die vollständige Aminosäure- und Nukleotidsequenz verschiedener Formen dieses Enzyms. Zur Veränderung der Cosubstrat-Nutzung in Richtung NADH wurden Mutationen für das Sequenzmotiv VLTGRNE vorgeschlagen, mit dem Ziel die Cosubstratnutzung von NADPH nach NADH zu verschieben. Die getesteten Mutanten hatten dabei die Befähigung zur Nutzung von NADP verloren: Mutanten mit erhöhter spezifischer Aktivität für den Cofaktor NADPH/NADP werden dort aber nicht vorgeschlagen.

Die bisher verfügbaren NADP/NADPH-nutzenden 12α-HSDHs besitzen jedoch für dieses Cofaktorsystem eine nicht völlig zufriedenstellende Aktivität.

Es ist daher Aufgabe der vorliegenden Erfindung neuartige 12α-HSDH-Mutanten bereitzustellen, die für den präparativen Einsatz bei der pharmazeutischen Wirkstoffsynthese in technischem Maßstab, wie z.B. bei der enzymkatalysierten Oxidation von Cholsäure zu 12-Ketochenodeoxycholsäure (12-Keto-CDCS), noch besser geeignet ist, indem sie eine verbesserte spezifische Aktivität für das Cofaktorsystem NADP/NADPH aufweisen und somit die Wirkstoffsynthese weiter fördern.

## Figurenbeschreibung

In den beiliegenden Figuren zeigt

Figur 1 einen vergrößerten Ausschnitt des Bereichs um G38 der 12α-HSDH. Dabei ist das $C_\alpha$-Atom des Glycinrestes (dunkel dargestellt) in unmittelbarer Nähe zu $N^3$ des Adenin-Teils von NADPH sowie zur Phosphatgruppe am C'2-Atom der Ribose im Adenosinteil von NADPH dargestellt;

Figur 2A die Auftragung der spezifischen Enzymaktivität gegen unterschiedliche eingesetzte Substratkonzentrationen bei konstanter Cofaktorkonzentration von 100 μM; B die Auftragung der Enzymaktivität gegen unterschiedliche eingesetzt Cofaktorkonzentrationen bei konstanter Substratkonzentration von 10 mM.

## Kurzfassung der Erfindung

Obige Aufgabe wurde überraschenderweise durch erstmalige Identifizierung derjenigen Aminosäuresequenzpositionen innerhalb der vollständigen Aminosäuresequenz des in *Clostridium* Gruppe P, strain C48-50 vorkommenden 12α-HSDH-Enzyms gelöst, die für eine Erhöhung der spezifischen Aktivität für den Cofaktor NADPH/NADP+ verantwortlich sind.

## Detaillierte Beschreibung der Erfindung

### 1. Spezielle Ausführungsformen

Ausgangspunkt für die erfindungsgemäß bereitzustellenden Mutanten sind insbesondere die rekombinant hergestellte 12α-Hydroxysteroiddehydrogenasen (12α-HSDHs) erhältlich aus *Clostridium* sp. wie in der WO 2009/118176 der vorliegenden Anmelderin beschrieben. Diese weisen ein Molekulargewicht, bestimmt durch SDS-Polyacrylamid-Gelelektrophorese (SDS-PAGE) unter reduzierenden Bedingungen im Bereich von mehr als etwa 26 kD, insbesondere mehr als etwa 26,5, wie etwa 27 bis 30 kD, und einem berechneten Molekulargewicht von mehr als etwa 29 kD, insbesondere etwa 29,1 bis 29, 5 kD, wie insbesondere 29,359 kD für HSDH_lang bzw etwa 27,8 für (HSDH_kurz) auf. Die Molekulargewichtangaben beziehen sich dabei auf das Molekulargewicht der Proteinuntereinheiten des Enzyms; ohne darauf beschränkt zu sein, besteht das native Protein z.B. aus 4, insbesondere in etwa gleich großen, solchen Untereinheiten.

Insbesondere ist ein derartiges Protein erhältlich aus *Clostridium* sp. group P strain 48-50 (DSM4029). Das Enzym kann z.B. in einer spezifischen Aktivität im Bereich von mehr als etwa 10, 15, 20 oder 25 U/mg, wie z.B. 20 bis 100 U/mg oder 25 bis 80 U/mg für das umzusetzende Hydroxysteroid bereitgestellt werden. Die Bestimmung der spezifischen Aktivität erfolgt dabei unter den im experimentellen Teil angegebenen Standardbedingungen.

Besondere Ausführungsformen der Erfindung sind insbesondere:

1. 12α-Hydroxysteroiddehydrogenase (12α-HSDH)-Mutante, welche wenigstens die stereospezifische enzymatische Oxidation eines 12-Hydroxysteroids zum korrespondierenden 12-Ketosteroid katalysiert, wobei die Mutante im Vergleich zum nicht-mutierten Enzym eine erhöhte spezifische Aktivität (U/mg) in Gegenwart des Cofaktors $NADP^+$ aufweist.

2. Mutante nach Ausführungsform 1, wobei spezifische Aktivität (U/mg), wie im folgenden experimentellen Teil bestimmt, in Gegenwart des Cofaktors $NADP^+$ um wenigsten 1, 5 oder 10%, wie z.B. um 1 bis 2.000%, oder 5 bis 1.000% oder 10 bis 500% oder 100 bis 400% erhöht ist, insbesondere aber wenigstens um das 1-fache, insbesondere um das 2- bis 10-fache erhöht ist.

3. 12α-Hydroxysteroiddehydrogenase (12α-HSDH)-Mutante, welche wenigstens die stereospezifische enzymatische Oxidation eines 12-Hydroxysteroids zum korrespondierenden 12-Ketosteroid katalysiert" wobei die Mutante wenigstens eine Mutation in derAminosäure-Sequenz gemäß SEQ ID NO: 2, 4, 6 oder 12 oder einer davon abgeleiteten Aminosäuresequenz mit wenigstens 60%, wenigstens 75%, oder wenigsten 85 %, wie z.B. 90, 91, 92, 93, 94, 95, 96, 97,98 oder 99%, Sequenzidentität aufweist.

4. Mutante nach einer der vorhergehenden Ausführungsformen, wobei die Mutante wenigstens eine Mutation im Sequenzmotiv VLTGRNE gemäß Position 35 bis 41 von SEQ ID NO:6 oder gemäß Position 34 bis 40 von SEQ ID NO:4; oder gemäß Position 47 bis 53 von SEQ ID NO:2; oder gemäß Position 35 bis 41 von SEQ ID NO:12 oder in dem korrespondierende Sequenzmotiv einer davon abgeleiteten Aminosäuresequenz mit wenigstens 60%, wenigstens 75%, oder wenigsten 85 %, wie z.B. 90, 91, 92, 93, 94, 95, 96, 97,98 oder 99%, Sequenzidentität aufweist.

5. Mutante nach Ausführungsform 4, ausgewählt unter den Einfachmutanten G→X, worin X für irgendeine natürliche, von G verschiedene Aminosäure steht, und insbesondere ausgewählt unter dem Mutanten G→A, G→S, G→V, G→P, G→N, G→D, G→E, G→Q, G→H, G→R, G→K und G→W oder den korrespondierenden Einfachmutanten einer davon abgeleiteten Aminosäuresequenz mit wenigstens 60%, wenigstens 75%, oder wenigsten 85 %, wie z.B. 90, 91, 92, 93, 94, 95, 96, 97,98 oder 99%, Sequenzidentität.

6. Mutante nach einer der Ausführungsformen 3 bis 5, wobei die Mutation ausgewählt ist unter G38A und G38S nach SEQ ID NO: 6.

7. Nukleinsäuresequenz kodierend für eine 12α-HSDH Mutante nach einer der vorhergehenden Ausführungsformen.

8. Expressionskassette, umfassend eine Nukleinsäuresequenz gemäß Ausführungsform 7 unter der genetischen Kontrolle wenigstens einer regulativen Nukleinsäuresequenz.

9. Vektor, umfassend wenigstens eine Expressionskassette nach Ausführungsform 8.

10. Rekombinanter Mikroorganismus, der wenigstens eine Nukleinsäuresequenz gemäß Ausführungsform 7 oder wenigstens eine Expressionskassette nach Ausführungsform 8 trägt oder mit wenigstens einem Vektor nach Ausführungsform 9 transformiert ist.

11. Verfahren zur enzymatischen Synthese von 12α -Hydroxysteroiden, wobei man das korrespondierende 12-Ketosteroid in Gegenwart einer 12α -HSDH Mutante gemäß der Definition in einer der Ausführungsformen 1 bis 6 umsetzt, und wenigstens ein gebildetes Reduktionsprodukt aus dem Reaktionsansatz gegebenenfalls isoliert.

12. Verfahren nach einer der Ausführungsformen 11 und 12, wobei die Reduktion in Gegenwart (und unter Verbrauch) von NADPH erfolgt.

13. Verfahren zur enzymatischen Oxidation von 12α-Hydroxysteroiden, wobei man das Hydroxysteroid in Gegenwart einer 12α -Hydroxysteroiddehydrogenase gemäß der Definition in einer der Ausführungsformen 1 bis 6 umsetzt, und ein gebildetes Oxidationsprodukt aus dem Reaktionsansatz gegebenenfalls isoliert.

14. Verfahren nach Ausführungsform 13, wobei das 12α -Hydroxysteroid CS oder ein Derivat davon, wie insbesondere ein Salz, Amid oder Alkylester, ist.

15. Verfahren nach einer der Ausführungsformen 12 und 14, wobei die Oxidation in Gegenwart (und unter Verbrauch) von $NADP^+$ erfolgt.

16. Verfahren nach einer der Ausführungsformen 12 bis 15, wobei die verbrauchten Redox-Äquivalente elektrochemisch oder enzymatisch regeneriert werden.

17. Verfahren nach Ausführungsform 16, wobei verbrauchtes NADP durch Kopplung mit einem NADP-regenerierenden Enzym ausgewählt unter einer NADP-Dehydrogenase und insbesondere einer Alkoholdehydrogenase (ADH), regeneriert wird.

18. Verfahren nach Ausführungsform 17, wobei das NADP-regenerierende Enzym ausgewählt ist unter natürlichen oder rekombinanten, isolierten oder angereicherten

a) Alkoholdehydrogenasen (EC 1.1.1.2) und
b) davon abgeleiteten funktionalen Äquivalenten

19. Verfahren zur Herstellung von Ursodesoxycholsäure (UDCS) der Formel (1)

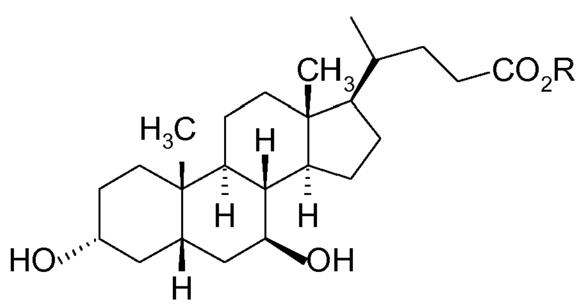

(1)

worin

R für Alkyl, $NR^1R^2$, H, ein Alkalimetallion oder $N(R^3)_4{}^+$steht, worin die Reste $R^3$ gleich oder verschieden sind und für H oder Alkyl stehen,

wobei man

a) eine Cholsäure (CS) der Formel (2)

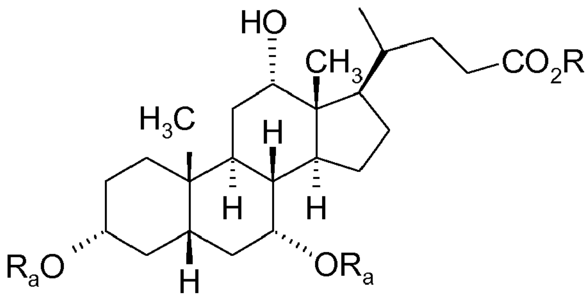

(2)

worin R die oben angegebenen Bedeutungen besitzen, und die Reste $R_a$ gleich oder verschieden sind und für H oder Acyl stehen, in Gegenwart einer 12α-Hydroxysteroiddehydrogenase-Mutante nach einer der Ausführungsformen 1 bis 6 zur korrespondierenden 12-Ketochenodesoxycholsäure (12-Keto CDCS) der Formel (3)

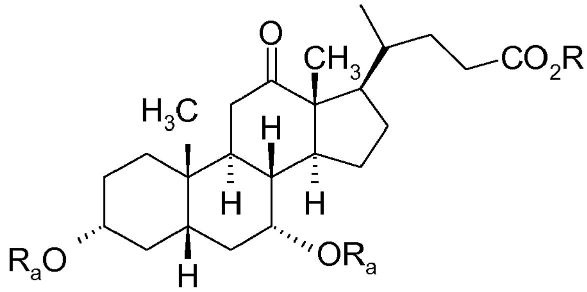

(3)

worin R und $R_a$ die oben angebebenen Bedeutungen besitzen, oxidiert und anschließend

b) 12-Keto-CDCS der Formel (3) durch Desoxygenierung zu Chenodesoxycholsäure (CDCS) der Formel (4)

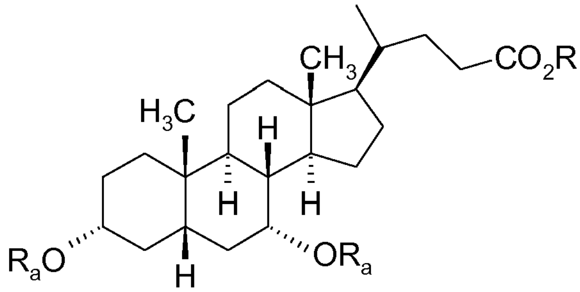

(4)

worin R und $R_a$ die oben angebebenen Bedeutungen besitzen, umsetzt und

c) CDCS der Formel (4) in Position 7 chemisch oxidiert zur 7-Keto-Lithocholsäure (KLCS) der Formel (5)

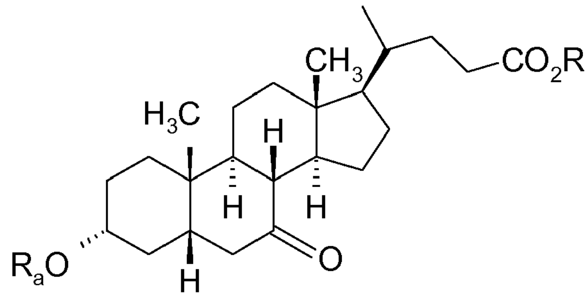

(5)

worin R und $R_a$ die oben angebebenen Bedeutungen besitzen; und

d) KLCS der Formel (5) reduziert und

e) das Reaktionsprodukt gegebenenfalls weiter aufreinigt.

Dabei kann, wenn $R_a$ für Acyl steht, diese Acylgruppe nach Durchführung der Reaktionsstufe b) oder d) gegebenenfalls abspalten werden.

20. Verfahren nach Ausführungsform 19, wobei Stufe a) in Gegenwart von $NADP^+$ erfolgt.

21. Verfahren nach Ausführungsform 20, wobei verbrauchtes $NADP^+$ elektrochemisch oder enzymatisch regeneriert werden.

22. Verfahren nach einer der Ausführungsformen 19 bis 21, wobei Stufe a) mit einer 12α-Hydroxysteroiddehydrogenase in immobilisierter Form erfolgt.

Die erfindungsgemäßen Mutationen sind gegebenenfalls weiter kombinierbar mit wenigstens einer die Produktinhibition verringernden Mutation im Bereich der die Substratbindungstasche des Enzyms bildenden Aminosäurereste; wie z.B. umfassend wenigstens die Mutation von Aminosäure Q(Gln) oder S(Ser), entsprechend Position 97 und /oder 99 von SEQ ID NO: 4, oder entsprechend Position 98 bzw 100 von SEQ ID NO: 6 oder 12; oder entsprechend Position 110 bzw. 112 gem. SEQ ID NO:2; insbesondere umfassend eine Mutation entsprechend Q97H in SEQ ID NO: 4, entsprechend Q98H in SEQ ID NO: 6 oder 12.

Weitere potentielle Aminosäuresubstituenten an Position 98 (bezogen auf SEQ ID NO: 6 oder 12) umfassen: A,N,D,C,E,G,H,M,S,T,V. Basierend auf dem Homologiemodell der erfindungsgemäßen HSDH wird angenommen, dass hier eine Substitution zu einer Schwächung der Carboxylbindung des Produkts führt. Daher wurde auch die benachbarte Position S100 (bezogen auf SEQ ID NO:6 oder 12) zu folgenden Aminosäuren mutiert: A,N,D,C,Q,E,G,H,M,T,V,K.

Eine Gruppe zusätzlicher Mutanten umfasst somit eine oder zwei Mutationen in Position 97 bzw. 99 (gemäß SEQ ID NO: 4) oder in Position 98 bzw. 100 (gemäß SEQ ID NO:6 oder 12) oder Position 110 bzw. 112 gemäß SEQ ID NO:2) ausgewählt unter:

Q → A,N,D,C,E,G,H,M,S,T,V;
S → A,N,D,C,Q,E,G,H,M,T,V,K.

Gegenstand der Erfindung sind insbesondere 12α-HSDH-Mutanten nach obiger Definition, erhältlich durch heterologe Expression wenigstens einer der oben beschriebenen12α-HSDH-kodierenden Nukleinsäuresequenzen, insbesondere solche rekombinant hergestellten Enzyme, exprimiert in einem nicht-pathogenen Mikroorganismus, wie z.B. exprimiert in einem Bakterium der Gattung Escherichia, insbesondere der Spezies E. coli.

In einer bevorzugten Ausführungsform obiger Redox-Reaktionen können die verbrauchten Redox-Äquivalente elektrochemisch oder enzymatisch regeneriert werden. Geeignete enzymatische Regenerationssysteme sind aus dem zitierten Stand der Technik bekannt. Nichtlimitierende Beispiele dafür geeigneter Enzyme sind Glutamatdehydrogenase, Alkoholdehydrogenase und Lactatdehydrogenase. Elektrochemische Regenerationsverfahren basieren z.B. auf Hydridorhodium-Redoxkatalysatoren, wie z.B. beschrieben in der WO-A-01/88172, worauf hiermit Bezug genommen wird.

In einer weiteren Ausgestaltung obiger Redoxreaktionen, können diese mit einer 12α-HSDH in immobilisierter Form erfolgen. Gegebenenfalls verwendete Enzyme für die Kofaktorregeneration können ebenfalls immobilisiert sein

Ein weiterer Gegenstand der Erfindung ist ein derartiger Bioreaktoren zur Durchführung obiger Redoxreaktionen, oder von solchen Teilreaktionsschritte im Rahmen eines synthetischen Gesamtprozesses.

Ein weiterer Gegenstand der Erfindung betrifft ein Verfahren zum qualitativen oder quantitativen Nachweis von 12-Ketosteroiden bzw. 12α-Hydroxysteroiden, wobei man das Steroid einer durch eine erfindungsgemäße 12α-HSDH-Mutanten katalysierten Redoxreaktion in Gegenwart von Redoxäquivalenten durchführt, eine Änderung in der Konzentration der Redoxäquivalente bestimmt und daraus den Gehalt an 12-Ketosteroiden bzw. 12α-Hydroxysteroiden qualitativ oder quantitativ ermittelt.

## 2. Allgemeine Definitionen

| Formel | Abkürzung | Chermischer Name |
|---|---|---|
| 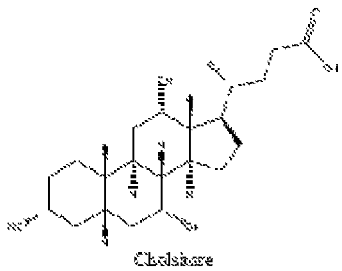 Cholsäure | CS | Cholsäure |
| 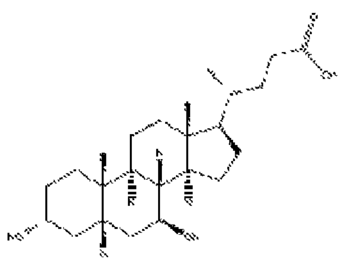 Ursodeoxycholsäure | UDCS | Ursodeoxycholsaure |
| 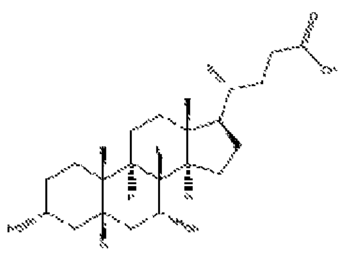 Chenodeoxycholsäure | CDCS | Chenodeoxycholsäure |
| 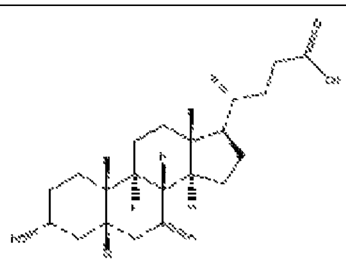 7-Keto-Lithocholsäure | 7-Keto-LCS | 7-Keto-Lithocholsäure |

(fortgesetzt)

| Formel | Abkürzung | Chermischer Name |
|---|---|---|
| 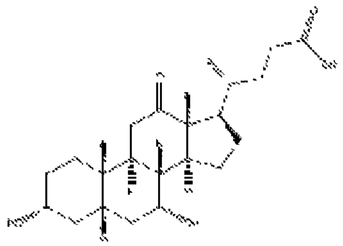 12-Keto-Chenodeoxycholsäure | 12-Keto-CDCS | 12-Keto-Chenodeoxychlosäure |

Werden keine anderen Angaben gemacht, so bezeichnet der Begriff "12α-HSDH" ein Dehydrogenaseenzym, welches wenigstens die stereospezifische Oxidation von Cholsäure zu 12-Keto-Chenodesoxycholsäure unter stöchiometrischem Verbrauch von $NAD^+$bzw. $NADP^+$ katalysiert. Das Enzym kann dabei ein natives bzw. rekombinant hergestelltes Enzym sein. Das Enzym kann grundsätzlich im Gemisch mit zellulären, wie z.B. Proteinverunreinigungen, vorzugsweise aber in Reinform vorliegen.

Unter einer "erhöhten spezifischen Aktivität in Gegenwart von $NADP^+$" versteht man eine Aktivität, ermittelt unter Bedingungen, welche die Bestimmung der spezifischen Aktivität eines Enzyms oder einer davon abgeleiteten Mutante für ein Substrat (hier 12-Hydroxisteroid-Substrat) erlauben, und gleichzeitig vermeiden, dass das Messergebnis möglicherweise durch eine zu geringe Cofaktor-Konzentration (hier $NADP^+$) verfälscht wird. Da die Einführung einer erfindungsgemäßen Mutation den Km-Wert für den Cofaktor verändern kann, sind bei Durchführung der Aktivitätsmessungen (für Mutante und nichtmutiertes Enzym) ausreichend hohe Cofaktor- Konzentrationen zu wählen, die keine Geschwindigkeits-Limitierung bewirken. Der Cofaktor sollte also bei derartigen Vergleichsmessungen im Überschuss vorliegen. Beispielsweise können derartige $NADP^+$-Konzentrationen im millimolaren Bereich, z.B. bei 1 bis 10mM oder 2 bis 5 mM, liegen. Eine "erhöhten spezifischen Aktivität in Gegenwart von $NADP^+$" kann auch als "erhöhte spezifische Aktivität in Gegenwart von $NADP^+$-Überschuss" bezeichnet werden.

Unter einer "Reinform" oder einem "reinen" oder "im wesentlichen reinen" Enzym versteht man erfindungsgemäß ein Enzym mit einem Reinheitsgrad von mehr als 80, vorzugsweise mehr als 90, insbesondere mehr als 95, und vor allem mehr als 99 Gew.-%, bezogen auf den Gesamtproteingehalt, bestimmt mit Hilfe üblicher Proteinnachweismethoden, wie .z.B. der Biuret-Methode oder dem Proteinnachweis nach Lowry.et al.(vgl Beschreibung in R.K. Scopes, Protein Purification, Springer Verlag, New York, Heidelberg, Berlin (1982)). Die spezifische Aktivität eines erfindungsgemäßen 12α-HSDH-Enzyms liegt dabei in dem oben angegebenen Bereich.

Unter einem "Redoxäquivalent" versteht man eine als Elektronendonor bzw. Elektronenakzeptor brauchbare, niedermolekulare organische Verbindung, wie beispielsweise Nikotinamidderivate wie $NAD^+$und $NADP^+$ bzw. deren reduzierte Formen NADH bzw. NADPH. Diese werden auch als "Cofaktor" bezeichnet.

Unter einer Verbindung eines speziellen Typs, wie z.B. einer "Cholsäure-Verbindung" oder einer "Ursodesoxycholsäure-Verbindung" versteht man insbesondere auch Derivate der zugrunde liegenden Ausgangsverbindung (wie z.B. Cholsäure oder Ursodesoxycholsäure).

Derartige Derivate umfassen "Salze", wie z.B. Alkalimetallsalze wie Lithium-, Natrium- und Kaliumsalze der Verbindungen; sowie Ammoniumsalze, wobei man unter einem Ammoniumsalz das $NH_4^+$-Salz bzw. solche Ammoniumsalze umfasst, worin wenigstens ein Wasserstoffatom durch einen $C_1$-$C_6$-Alkylrest ersetzt sein kann. Typische Alkylreste sind insbesondere $C_1$-$C_4$-Alkylreste, wie Methyl, Ethyl, n- oder i-Propyl-, n-, sec- oder tert-Butyl, sowie n-Pentyl und n-Hexyl und die ein- oder mehrfach verzweigten Analoga davon

"Alkylester" erfindungsgemäßer Verbindungen sind insbesondere Niedrigalkyl-Ester, wie z.B. $C_1$-$C_6$-Alkylester. Als nicht limitierende Beispiele sind zu nennen Methyl-, Ethyl-, n- oder i-Propyl-, n-, sec- oder tert-Butylester, oder längerkettige Ester, wie z.B. n-Pentyl- und n-Hexylester sowie die ein- oder mehrfach verzweigten Analoga davon.

"Amide" sind insbesondere Umsetzungsprodukte erfindungsgemäßer Säuren mit Ammoniak oder primären oder sekundären Monoaminen. Derartige Amine sind beispielsweise Mono-oder Di-$C_1$-$C_6$-Alkyl-monoamine, wobei die Alkylreste unabhängig voneinander gegebenenfalls weiter substituiert sein können, wie z.B. durch Carboxy-, Hydroxy-, Halogen (wie F, Cl, Br, J)-, Nitro- und Sulfonatgruppen.

Erfindungsgemäße "Acylgruppen" sind insbesondere nichtaromatische Gruppen mit 2 bis 4 Kohlenstoffatomen, wie z.B. Acetyl, Propionyl und Butyryl, sowie aromatische Gruppen mit gegebenenfalls substituiertem einkernigen aromatischem Ring, wobei geeignete Substituenten z.B. ausgewählt sind unter Hydroxy-, Halogen (wie F, Cl, Br, J)-, Nitro- und $C_1$-$C_6$-Alkylgruppen, wie z.B. Benzoyl oder Toluoyl.

Die erfindungsgemäß eingesetzten bzw. hergestellten Hydroxysteroidverbindungen, wie Cholsäure, Glykocholsäure, Taurocholsäure Ursodesoxycholsäure, 12-Keto-Chenodesoxycholsäure, Chenodesoxycholsäure und 7-Keto-Lithocholsäure können in stereoisomerenreiner Reinform oder im Gemisch mit anderen Stereoisomeren im erfindungsgemäßen Verfahren eingesetzt oder daraus erhalten werden. Vorzugsweise werden jedoch die eingesetzten bzw. die hergestellten Verbindungen in im Wesentlichen stereoisomerenreiner Form eingesetzt bzw. isoliert.

Unter einer "Immobilisierung" versteht man erfindungsgemäß die kovalente oder nichtkovalente Bindung eines erfindungsgemäß verwendeten Biokatalysators, wie z.B. einer 12α-HSDH an einem festen, d.h. in dem umgebenden flüssigen Medium im Wesentlichen unlöslichen, Trägermaterial.

"Produktinhibition" der 12α-HSDH beschreibt die Verringerung der enzymatischen Aktivität in Gegenwart eines bei einer durch das Enzym katalysierten enzymatischen Umsetzung gebildeten Produkts. Bei Umsetzung zu CS ist so z.B. eine Inhibition durch 12-Keto-CDCS zu beobachten. Eine "Verringerung der Produktinhibition" beschreibt die verringerte prozentuale Abnahme der Enzymaktivität einer erfindungsgemäßen Enzymmutante im Vergleich zu einem Referenzsystem, wie z.B. dem nativen HSDH-Enzym, jeweils bezogen auf die bei 0% Umsatz (entsprechend 5mM CS) ermittelte Enzymaktivität als 100%-Aktivitätswert. Diese Verringerung kann, wie im experimentellen Teil, bzw. in der Legende zu Figur 9 beschrieben, bestimmt werden. Erfindungsgemäße Verringerungen der Produktinhibition können auch über das Verhältnis der Restaktivitäten von Mutante zu Referenzenzym jeweils bestimmt bei gleichem prozentualen Umsatz, ausgedrückt werden. So kann die erfindungsgemäße Mutante eine um den Faktor 1,1 bis 100, wie z.B 1,5 bis 20 oder 2 bis 10 erhöhte Aktivität besitzen.

### 3. Weitere Ausgestaltungen der Erfindung

### 3.1 Proteine

Die vorliegende Erfindung ist nicht auf die konkret offenbarten Mutanten mit 12α-HSDH-Aktivität beschränkt, sondern erstreckt sich vielmehr auch auf funktionale Äquivalente davon.

"Funktionale Äquivalente" oder Analoga der konkret offenbarten Enzymmutanten sind im Rahmen der vorliegenden Erfindung davon verschiedene Polypeptide, welche weiterhin die gewünschte biologische Aktivität, wie z.B. 12α-HSDH-Aktivität, besitzen, und weiterhin das erfindungsgemäße Mutationsmuster zeigen.

So versteht man beispielsweise unter "funktionalen Äquivalenten" Enzyme, die im verwendeten Test auf 12α-HSDH-Aktivität unter identischen Bedingungen, eine um mindestens 1 % , wie z.B. mindestens 10% oder 20 %, wie z.B. mindestens 50 % oder 75% oder 90 % höhere oder niedrigere Aktivität eines Enzyms, umfassend eine hierin definierte Aminosäuresequenz aufweisen. Funktionale Äquivalente sind außerdem vorzugsweise zwischen pH 4 bis 11 stabil und besitzen vorteilhaft ein pH-Optimum in einem Bereich von pH 6 bis 10, wie insbesondere 8,5 bis 9,5, sowie ein Temperaturoptimum im Bereich von 15°C bis 80°C oder 20°C bis 70°C, wie z.B. etwa 45 bis 60°C oder etwa 50 bis 55°C. Insbesondere sollte dabei der für die Umsetzung erforderliche Cofaktor im Überschuß , d.h. nicht in Geschwindigkeits-limitierenden Menge vorliegen.

Die 12α-HSDH -Aktivität kann mit Hilfe verschiedener bekannter Tests nachgewiesen werden. Ohne darauf beschränkt zu sein, sei ein Test unter Verwendung eines Referenzsubstrates, wie z. B. Cholsäure, unter standardisierten Bedingungen wie im experimentellen Teil definiert, zu nennen.

Unter "funktionalen Äquivalenten" versteht man erfindungsgemäß insbesondere auch "Mutanten", welche in wenigstens einer Sequenzposition der oben genannten Aminosäuresequenzen eine andere als die konkret genannte Aminosäure aufweisen aber trotzdem eine der oben genannten biologischen Aktivitäten besitzen. "Funktionale Äquivalente" umfassen somit die durch eine oder mehrere Aminosäure-Additionen, -Substitutionen, -Deletionen und/oder -Inversionen erhältlichen Mutanten, wobei die genannten Veränderungen in jeglicher Sequenzposition auftreten können, solange sie zu einer Mutante mit dem erfindungsgemäßen Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d.h. beispielsweise gleiche Substrate mit unterschiedlicher Geschwindigkeit umgesetzt werden. Beispiele für geeignete Aminosäuresubstitutionen sind in folgender Tabelle zusammengefasst:

| Ursprünglicher Rest | Beispiele der Substitution |
|---|---|
| Ala | Ser |
| Arg | Lys |
| Asn | Gln; His |
| Asp | Glu |
| Cys | Ser |
| Gln | Asn |
| Glu | Asp |
| Gly | Pro |

(fortgesetzt)

| Ursprünglicher Rest | Beispiele der Substitution |
|---|---|
| His | Asn; Gln |
| Ile | Leu; Val |
| Leu | Ile; Val |
| Lys | Arg ; Gln; Glu |
| Met | Leu ; Ile |
| Phe | Met; Leu; Tyr |
| Ser | Thr |
| Thr | Ser |
| Trp | Tyr |
| Tyr | Trp ; Phe |
| Val | Ile; Leu |

"Funktionale Äquivalente" im obigen Sinne sind auch "Präkursoren" der beschriebenen Polypeptide sowie "funktionale Derivate" und "Salze" der Polypeptide.

"Präkursoren" sind dabei natürliche oder synthetische Vorstufen der Polypeptide mit oder ohne der gewünschten biologischen Aktivität.

Unter dem Ausdruck "Salze" versteht man sowohl Salze von Carboxylgruppen als auch Säureadditionssalze von Aminogruppen der erfindungsgemäßen Proteinmoleküle. Salze von Carboxylgruppen können in an sich bekannter Weise hergestellt werden und umfassen anorganische Salze, wie zum Beispiel Natrium-, Calcium-, Ammonium-, Eisen- und Zinksalze, sowie Salze mit organischen Basen, wie zum Beispiel Aminen, wie Triethanolamin, Arginin, Lysin, Piperidin und dergleichen. Säureadditionssalze, wie zum Beispiel Salze mit Mineralsäuren, wie Salzsäure oder Schwefelsäure und Salze mit organischen Säuren, wie Essigsäure und Oxalsäure sind ebenfalls Gegenstand der Erfindung.

"Funktionale Derivate" erfindungsgemäßer Polypeptide können an funktionellen Aminosäure-Seitengruppen oder an deren N- oder C-terminalen Ende mit Hilfe bekannter Techniken ebenfalls hergestellt werden. Derartige Derivate umfassen beispielsweise aliphatische Ester von Carbonsäuregruppen, Amide von Carbonsäuregruppen, erhältlich durch Umsetzung mit Ammoniak oder mit einem primären oder sekundären Amin; N-Acylderivate freier Aminogruppen, hergestellt durch Umsetzung mit Acylgruppen; oder O-Acylderivate freier Hydroxygruppen, hergestellt durch Umsetzung mit Acylgruppen.

"Funktionale Äquivalente" umfassen natürlich auch Polypeptide welche aus anderen Organismen zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Enzyme ermitteln.

"Funktionale Äquivalente" umfassen ebenfalls Fragmente, vorzugsweise einzelne Domänen oder Sequenzmotive, der erfindungsgemäßen Polypeptide, welche z.B. die gewünschte biologische Funktion aufweisen.

"Funktionale Äquivalente" sind außerdem Fusionsproteine, welche eine der oben genannten Polypeptidsequenzen oder davon abgeleitete funktionale Äquivalente und wenigstens eine weitere, davon funktionell verschiedene, heterologe Sequenz in funktioneller N- oder C-terminaler Verknüpfung (d.h. ohne gegenseitigen wesentliche funktionelle Beeinträchtigung der Fusionsproteinteile) aufweisen. Nichtlimitierende Beispiele für derartige heterologe Sequenzen sind z.B. Signalpeptide, Histidin-Anker oder Enzyme.

Erfindungsgemäß mit umfasste "funktionale Äquivalente" sind Homologe zu den konkret offenbarten Proteinen. Diese besitzen wenigstens 60 %, vorzugsweise wenigstens 75% ins besondere wenigsten 85 %, wie z.B. 90, 91, 92, 93, 94, 95, 96, 97,98 oder 99%, Homologie (bzw. Identität) zu einer der konkret offenbarten Aminosäuresequenzen, berechnet nach dem Algorithmus von Pearson und Lipman, Proc. Natl. Acad, Sci. (USA) 85(8), 1988, 2444-2448. Eine prozentuale Homologie bzw. Identität eines erfindungsgemäßen homologen Polypeptids bedeutet insbesondere prozentuale Identität der Aminosäurereste bezogen auf die Gesamtlänge einer der hierin konkret beschriebenen Aminosäuresequenzen.

Die prozentualen Identitätswerte können auch anhand von BLAST Alignments, Algorithmus blastp (protein-protein BLAST), oder durch Anwendung der unten angegebenen Clustal Einstellungen ermittelt werden.

Im Falle einer möglichen Proteinglykosylierung umfassen erfindungsgemäße "funktionale Äquivalente" Proteine des oben bezeichneten Typs in deglykosylierter bzw. glykosylierter Form sowie durch Veränderung des Glykosylierungsmusters erhältliche abgewandelte Formen.

Homologe der erfindungsgemäßen Proteine oder Polypeptide können durch Mutagenese erzeugt werden, z.B. durch Punktmutation, Verlängerung oder Verkürzung des Proteins.

Homologe der erfindungsgemäßen Proteine können durch Screening kombinatorischer Banken von Mutanten, wie z.B. Verkürzungsmutanten, identifiziert werden. Beispielsweise kann eine variegierte Bank von Protein-Varianten

durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen kodieren. Verfahren zur Synthese degenerierter Oligonukleotide sind dem Fachmann bekannt (z.B. Narang, S.A. (1983) Tetrahedron 39:3; Itakura et al. (1984) Annu. Rev. Biochem. 53:323; Itakura et al., (1984) Science 198:1056; Ike et al. (1983) Nucleic Acids Res. 11:477).

Im Stand der Technik sind mehrere Techniken zum Screening von Genprodukten kombinatorischer Banken, die durch Punktmutationen oder Verkürzung hergestellt worden sind, und zum Screening von cDNA-Banken auf Genprodukte mit einer ausgewählten Eigenschaft bekannt. Diese Techniken lassen sich an das schnelle Screening der Genbanken anpassen, die durch kombinatorische Mutagenese erfindungsgemäßer Homologer erzeugt worden sind. Die am häufigsten verwendeten Techniken zum Screening großer Genbanken, die einer Analyse mit hohem Durchsatz unterliegen, umfassen das Klonieren der Genbank in replizierbare Expressionsvektoren, Transformieren der geeigneten Zellen mit der resultierenden Vektorenbank und Exprimieren der kombinatorischen Gene unter Bedingungen, unter denen der Nachweis der gewünschten Aktivität die Isolation des Vektors, der das Gen kodiert, dessen Produkt nachgewiesen wurde, erleichtert. Recursive-Ensemble-Mutagenese (REM), eine Technik, die die Häufigkeit funktioneller Mutanten in den Banken vergrößert, kann in Kombination mit den Screeningtests verwendet werden, um Homologe zu identifizieren (Arkin und Yourvan (1992) PNAS 89:7811-7815; Delgrave et al. (1993) Protein Engineering 6(3):327-331).

### 3.2 Nukleinsäuren und Konstrukte

#### 3.2.1 Nukleinsäuren

Gegenstand der Erfindung sind auch Nukleinsäuresequenzen, die für ein Enzym mit 12α-HSDH-Aktivität kodieren.

Die vorliegende Erfindung betrifft auch Nukleinsäuren mit einem bestimmten Identitätsgrad zu den hierin beschriebenen konkreten Sequenzen.

Unter "Identität" zwischen zwei Nukleinsäuren wird die Identität der Nukleotide über die jeweils gesamte Nukleinsäurelänge verstanden, insbesondere die Identität, die durch Vergleich mit Hilfe der Vector NTI Suite 7.1 Software der Firma Informax (USA) unter Anwendung der Clustal Methode (Higgins DG, Sharp PM. Fast and sensitive multiple sequence alignments on a microcomputer. Comput Appl. Biosci. 1989 Apr;5(2):151-1) unter Einstellung folgender Parameter berechnet wird:

| | |
|---|---|
| Multiple alignment parameters: | |
| Gap opening penalty | 10 |
| Gap extension penalty | 10 |
| Gap separation penalty range | 8 |
| Gap separation penalty | off |
| % identity for alignment delay | 40 |
| Residue specific gaps | off |
| Hydrophilic residue gap | off |
| Transition weighing | 0 |
| | |
| Pairwise alignment parameter: | |
| FAST algorithm | on |
| K-tuple size | 1 |
| Gap penalty | 3 |
| Window size | 5 |
| Number of best diagonals | 5 |

Alternativ dazu kann die Identität auch nach Chenna, Ramu, Sugawara, Hideaki, Ko-ike,Tadashi, Lopez, Rodrigo, Gibson, TobyJ, Higgins, Desmond G, Thompson, Julie D. Multiple sequence alignment with the Clustal series of programs. (2003) Nucleic Acids Res 31 (13):3497-500, gemäß Internetadresse: http://www.ebi.ac.uk/Tools/clustalw/index.html# und mit den folgenden Parametern bestimmt werden:

| | |
|---|---|
| DNA Gap Open Penalty | 15.0 |
| DNA Gap Extension Penalty | 6.66 |
| DNA Matrix | Identity |
| Protein Gap Open Penalty | 10.0 |
| Protein Gap Extension Penalty | 0.2 |
| Protein matrix | Gonnet |
| Protein/DNA ENDGAP | -1 |
| Protein/DNA GAPDIST | 4 |

Alle hierin erwähnten Nukleinsäuresequenzen (einzel- und doppelsträngige DNA- und RNA-Sequenzen, wie z.B. cDNA und mRNA) sind in an sich bekannter Weise durch chemische Synthese aus den Nukleotidbausteinen, wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seiten 896-897) erfolgen. Die Anlagerung synthetischer Oligonukleotide und Auffüllen von Lücken mit Hilfe des Klenow-Fragmentes der DNA-Polymerase und Ligationsreaktionen sowie allgemeine Klonierungsverfahren werden in Sambrook et al. (1989), Molecular Cloning: A laboratory manual, Cold Spring Harbor Laboratory Press, beschrieben.

Gegenstand der Erfindung sind auch Nukleinsäuresequenzen (einzel- und doppelsträngige DNA- und RNA-Sequenzen, wie z.B. cDNA und mRNA), kodierend für eines der obigen Polypeptide und deren funktionalen Äquivalente, welche z.B. unter Verwendung künstlicher Nukleotidanaloga zugänglich sind.

Die Erfindung betrifft sowohl isolierte Nukleinsäuremoleküle, welche für erfindungsgemäße Polypeptide bzw. Proteine oder biologisch aktive Abschnitte davon kodieren, als auch Nukleinsäurefragmente, die z.B. zur Verwendung als Hybridisierungssonden oder Primer zur Identifizierung oder Amplifizierung von erfindungsgemäßer kodierenden Nukleinsäuren verwendet werden können.

Die erfindungsgemäßen Nukleinsäuremoleküle können zudem untranslatierte Sequenzen vom 3'- und/oder 5'-Ende des kodierenden Genbereichs enthalten

Die Erfindung umfasst weiterhin die zu den konkret beschriebenen Nukleotidsequenzen komplementären Nukleinsäuremoleküle oder einen Abschnitt davon.

Die erfindungsgemäßen Nukleotidsequenzen ermöglichen die Erzeugung von Sonden und Primern, die zur Identifizierung und/oder Klonierung von homologen Sequenzen in anderen Zelltypen und Organismen verwendbar sind. Solche Sonden bzw. Primer umfassen gewöhnlich einen Nukleotidsequenzbereich, der unter "stringenten" Bedingungen (siehe unten) an mindestens etwa 12, vorzugsweise mindestens etwa 25, wie z.B. etwa 40, 50 oder 75 aufeinanderfolgende Nukleotide eines Sense-Stranges einer erfindungsgemäßen Nukleinsäuresequenz oder eines entsprechenden Antisense-Stranges hybridisiert.

Ein "isoliertes" Nukleinsäuremolekül wird von anderen Nukleinsäuremolekülen abgetrennt, die in der natürlichen Quelle der Nukleinsäure zugegen sind und kann überdies im wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

Ein erfindungsgemäßes Nukleinsäuremolekül kann mittels molekularbiologischer Standard-Techniken und der erfindungsgemäß bereitgestellten Sequenzinformation isoliert werden. Beispielsweise kann cDNA aus einer geeigneten cDNA-Bank isoliert werden, indem eine der konkret offenbarten vollständigen Sequenzen oder ein Abschnitt davon als Hybridisierungssonde und Standard-Hybridisierungstechniken (wie z.B. beschrieben in Sambrook, J., Fritsch, E.F. und Maniatis, T. Molecular Cloning: A Laboratory Manual. 2. Aufl., Cold Spring Harbor Laboratory, Cold Spring Harbor Laboratory Press, Cold Spring Harbor, NY, 1989) verwendet werden. Überdies lässt sich ein Nukleinsäuremolekül, umfassend eine der offenbarten Sequenzen oder ein Abschnitt davon, durch Polymerasekettenreaktion isolieren, wobei die Oligonukleotidprimer, die auf der Basis dieser Sequenz erstellt wurden, verwendet werden. Die so amplifizierte Nukleinsäure kann in einen geeigneten Vektor kloniert werden und durch DNA-Sequenzanalyse charakterisiert werden. Die erfindungsgemäßen Oligonukleotide können ferner durch Standard-Syntheseverfahren, z.B. mit einem automatischen DNA-Synthesegerät, hergestellt werden.

Erfindungsgemäße Nukleinsäuresequenzen oder Derivate davon, Homologe oder Teile dieser Sequenzen, lassen sich beispielsweise mit üblichen Hybridisierungsverfahren oder der PCR-Technik aus anderen Bakterien, z.B. über genomische oder cDNA-Banken, isolieren. Diese DNA-Sequenzen hybridisieren unter Standardbedingungen mit den erfindungsgemäßen Sequenzen.

Unter "hybridisieren" versteht man die Fähigkeit eines Poly- oder Oligonukleotids an eine nahezu komplementäre Sequenz unter Standardbedingungen zu binden, während unter diesen Bedingungen unspezifische Bindungen

zwischen nicht-komplementären Partnern unterbleiben. Dazu können die Sequenzen zu 90-100% komplementär sein. Die Eigenschaft komplementärer Sequenzen, spezifisch aneinander binden zu können, macht man sich beispielsweise in der Northern- oder Southern-Blot-Technik oder bei der Primerbindung in PCR oder RT-PCR zunutze.

Zur Hybridisierung werden vorteilhaft kurze Oligonukleotide der konservierten Bereiche verwendet. Es können aber auch längere Fragmente der erfindungsgemäßen Nukleinsäuren oder die vollständigen Sequenzen für die Hybridisierung verwendet werden. Je nach der verwendeten Nukleinsäure (Oligonukleotid, längeres Fragment oder vollständige Sequenz) oder je nachdem welche Nukleinsäureart DNA oder RNA für die Hybridisierung verwendet werden, variieren diese Standardbedingungen. So liegen beispielsweise die Schmelztemperaturen für DNA:DNA-Hybride ca 10 ˚C niedriger als die von DNA:RNA-Hybriden gleicher Länge.

Unter Standardbedingungen sind beispielsweise je nach Nukleinsäure Temperaturen zwischen 42 und 58 ˚C in einer wässrigen Pufferlösung mit einer Konzentration zwischen 0,1 bis 5 x SSC (1 X SSC = 0,15 M NaCl, 15 mM Natriumcitrat, pH 7,2) oder zusätzlich in Gegenwart von 50% Formamid wie beispielsweise 42 ˚C in 5 x SSC, 50% Formamid zu verstehen. Vorteilhafterweise liegen die Hybridisierungsbedingungen für DNA:DNA-Hybride bei 0,1 x SSC und Temperaturen zwischen etwa 20 ˚C bis 45 ˚C, bevorzugt zwischen etwa 30 ˚C bis 45 ˚C. Für DNA:RNA-Hybride liegen die Hybridisierungsbedingungen vorteilhaft bei 0,1 x SSC und Temperaturen zwischen etwa 30 ˚C bis 55 ˚C, bevorzugt zwischen etwa 45 ˚C bis 55 ˚C. Diese angegebenen Temperaturen für die Hybridisierung sind beispielhaft kalkulierte Schmelztemperaturwerte für eine Nukleinsäure mit einer Länge von ca. 100 Nukleotiden und einem G + C-Gehalt von 50 % in Abwesenheit von Formamid. Die experimentellen Bedingungen für die DNA-Hybridisierung sind in einschlägigen Lehrbüchern der Genetik, wie beispielsweise Sambrook et al., "Molecular Cloning", Cold Spring Harbor Laboratory, 1989, beschrieben und lassen sich nach dem Fachmann bekannten Formeln beispielsweise abhängig von der Länge der Nukleinsäuren, der Art der Hybride oder dem G + C-Gehalt berechnen. Weitere Informationen zur Hybridisierung kann der Fachmann folgenden Lehrbüchern entnehmen: Ausubel et al. (eds), 1985, Current Protocols in Molecular Biology, John Wiley & Sons, New York; Hames and Higgins (eds), 1985, Nucleic Acids Hybridization: A Practical Approach, IRL Press at Oxford University Press, Oxford; Brown (ed), 1991, Essential Molecular Biology: A Practical Approach, IRL Press at Oxford University Press, Oxford.

Die "Hybridisierung" kann insbesondere unter stringenten Bedingungen erfolgen. Solche Hybridisierungsbedingungen sind beispielsweise bei Sambrook, J., Fritsch, E.F., Maniatis, T., in: Molecular Cloning (A Laboratory Manual), 2. Auflage, Cold Spring Harbor Laboratory Press, 1989, Seiten 9.31-9.57 oder in Current Protocols in Molecular Biology, John Wiley & Sons, N.Y. (1989), 6.3.1-6.3.6 beschrieben.

Unter "stringenten" Hybridisierungs-Bedingungen werden insbesondere verstanden: Die Inkubation bei 42˚C über Nacht in einer Lösung bestehend aus 50 % Formamid, 5 x SSC (750 mM NaCl, 75 mM Tri-Natrium-citrat), 50 mM Natrium Phosphat (pH7,6), 5x Denhardt Lösung, 10% Dextransulfat und 20 g/ml denaturierte, gescheerte Lachsspermien-DNA, gefolgt von einem Waschschritt der Filter mit 0,1x SSC bei 65˚C.

Gegenstand der Erfindung sind auch Derivate der konkret offenbarten oder ableitbaren Nukleinsäuresequenzen.

So können weitere erfindungsgemäße Nukleinsäuresequenzen z.B. von SEQ ID NO:1, 3, 5 oder 11 abgeleitet sein und sich davon durch Addition, Substitution, Insertion oder Deletion einzelner oder mehrerer Nukleotide unterscheiden, aber weiterhin für Polypeptide mit dem gewünschten Eigenschaftsprofil kodieren.

Erfindungsgemäß umfasst sind auch solche Nukleinsäuresequenzen, die sogenannte stumme Mutationen umfassen oder entsprechend der Codon-Nutzung eins speziellen Ursprungs-oder Wirtsorganismus, im Vergleich zu einer konkret genannten Sequenz verändert sind, ebenso wie natürlich vorkommende Varianten, wie z.B. Spleißvarianten oder Allelvarianten, davon.

Gegenstand sind ebenso durch konservative Nukleotidsubstutionen (d.h. die betreffende Aminosäure wird durch eine Aminosäure gleicher Ladung, Größe, Polarität und/oder Löslichkeit ersetzt) erhältliche Sequenzen.

Die Anpassung der Nukleinsäuresequenz an die Codon-Nutzung eines Organismus kann dabei nach üblichen Methoden erfolgen, wie z.B. unter zugänglich unter: http://slam.bs.jhmi.edu/cgi-bin/gd/gdRevTrans.cgi. Gegenstand der Erfindung sind auch solche N.S. Sequenzen.

Gegenstand der Erfindung sind auch die durch Sequenzpolymorphismen von den konkret offenbarten Nukleinsäuren abgeleiteten Moleküle. Diese genetischen Polymorphismen können zwischen Individuen innerhalb einer Population aufgrund der natürlichen Variation existieren. Diese natürlichen Variationen bewirken üblicherweise eine Varianz von 1 bis 5 % in der Nukleotidsequenz eines Gens.

Unter Derivaten der erfindungsgemäßen Nukleinsäuresequenz mit der Sequenz SEQ ID NO: 1, 3, 5 oder11 sind beispielsweise Allelvarianten zu verstehen, die mindestens 60 % Homologie auf der abgeleiteten Aminosäureebene, bevorzugt mindestens 80 % Homologie, ganz besonders bevorzugt mindestens 90 % Homologie über den gesamten Sequenzbereich aufweisen (bezüglich Homologie auf Aminosäureebene sei auf obige Ausführungen zu den Polypeptiden verwiesen auf). Über Teilbereiche der Sequenzen können die Homologien vorteilhaft höher liegen.

Weiterhin sind unter Derivaten auch Homologe der erfindungsgemäßen Nukleinsäuresequenzen, insbesondere der SEQ ID NO: 1, 3, 5 und 11, beispielsweise pilzliche oder bakterielle Homologe, verkürzte Sequenzen, Einzelstrang-DNA oder RNA der kodierenden und nichtkodierenden DNA-Sequenz, zu verstehen. So besitzen z.B. Homologe zur der SEQ ID NO: 1, 3, 5 oder 11 auf DNA-Ebene eine Homologie von mindestens 40 %, bevorzugt von mindestens 60 %, besonders bevorzugt von mindestens 70 %, ganz besonders bevorzugt von mindestens 80 % über den gesamten in SEQ ID NO: 7 angegebenen DNA-Bereich.

Außerdem sind unter Derivaten beispielsweise Fusionen mit Promotoren zu verstehen. Die Promotoren, die den angegebenen Nukleotidsequenzen vorgeschalten sind, können durch wenigstens einen Nukleotidaustausch, wenigstens eine Insertionen, Inversionen und/oder Deletionen verändert sein, ohne dass aber die Funktionalität bzw. Wirksamkeit der Promotoren beeinträchtigt sind. Des weiteren können die Promotoren durch Veränderung ihrer Sequenz in ihrer Wirksamkeit erhöht oder komplett durch wirksamere Promotoren auch artfremder Organismen ausgetauscht werden.

Dem Fachmann sind darüber hinaus Verfahren zur Erzeugung funktionaler Mutanten bekannt.

Je nach verwendeter Technik kann der Fachmann völlig zufällige oder auch gezieltere Mutationen in Gene oder auch nicht codierende Nukleinsäurebereiche (die beispielsweise für die Regulation der Expression wichtig sind) einbringen und anschließend Genbanken erstellen. Die dazu erforderlichen molekularbiologischen Methoden sind dem Fachmann bekannt und beispielsweise beschrieben in Sambrook und Russell, Molecular Cloning. 3. Edition, Cold Spring Harbor Laboratory Press 2001.

Methoden zur Veränderung von Genen und somit zur Veränderung der durch diese codierten Protein sind dem Fachmann seit langem geläufig, wie beispielsweise

- die ortsspezifische Mutagenese, bei der gezielt einzelne oder mehrere Nukleotide eines Gens ausgetauscht werden (Trower MK (Hrsg.) 1996; In vitro mutagenesis protocols. Humana Press, New Jersey),
- die Sättigungsmutagenese, bei der an jeder beliebigen Stelle eines Gens ein Codon für eine beliebige Aminosäure ausgetauscht oder hinzugefügt werden kann (Kegler-Ebo DM, Docktor CM, DiMaio D (1994) Nucleic Acids Res 22: 1593; Barettino D, Feigenbutz M, Valcärel R, Stunnenberg HG (1994) Nucleic Acids Res 22:541; Barik S (1995) Mol Biotechnol 3:1),
- die fehleranfällige Polymerase-Kettenreaktion (error-prone PCR), bei der Nukleotidsequenzen durch fehlerhaft arbeitende DNA-Polymerasen mutiert werden (Eckert KA, Kunkel TA (1990) Nucleic Acids Res 18:3739);
- das Passagieren von Genen in Mutator-Stämmen, in denen beispielsweise aufgrund defekter DNA-Reperaturmechanismen eine erhöhte Mutationsrate von Nukleotidsequenzen auftritt (Greener A, Callahan M, Jerpseth B (1996) An efficient random mutagenesis technique using an E.coli mutator strain. In: Trower MK (Hrsg.) In vitro mutagenesis protocols. Humana Press, New Jersey), oder
- das DNA-Shuffling, bei dem ein Pool aus nahe verwandten Genen gebildet und verdaut wird und die Bruchstücke als Templates für eine Polymerase-Kettenreaktion verwendet werden, bei der durch wiederholte Strangtrennung und Wiederannäherung letztendlich Mosaikgene voller Länge erzeugt werden (Stemmer WPC (1994) Nature 370: 389; Stemmer WPC (1994) Proc Natl Acad Sci USA 91:10747).

Unter Anwendung der sogenannten gerichteten Evolution ("directed evolution"; beschrieben unter anderem in Reetz MT und Jaeger K-E (1999), Topics Curr Chem 200:31; Zhao H, Moore JC, Volkov AA, Arnold FH (1999), Methods for optimizing industrial enzymes by directed evolution, In: Demain AL, Davies JE (Hrsg.) Manual of industrial microbiologyand biotechnology. American Society for Microbiology) kann der Fachmann auch in gezielter Weise und auch in großem Maßstab funktionale Mutanten erzeugen. Dabei werden in einem ersten Schritt zunächst Genbanken der jeweiligen Proteine erzeugt, wobei beispielsweise die oben angegebenen Methoden zur Anwendung kommen können. Die Genbanken werden auf geeignete Weise exprimiert, beispielsweise durch Bakterien oder durch Phagen-Display-Systeme.

Die betreffenden Gene von Wirtsorganismen, die funktionale Mutanten mit Eigenschaften exprimieren, welche den gewünschten Eigenschaften weitgehend entsprechen, können einer weiteren Mutationsrunde unterworfen werden. Die Schritte der Mutation und der Selektion oder des Screening können iterativ solange wiederholt werden, bis die vorliegenden funktionalen Mutanten die gewünschten Eigenschaften in ausreichendem Maße aufweisen. Durch diese iterative Arbeitsweise können stufenweise eine begrenzte Anzahl von Mutationen , wie z.B. 1 bis 5 Mutationen, vorgenommen und auf deren Einfluss auf die betreffende Enzymeigenschaft bewertet und selektiert werden. Die selektierte Mutante kann dann in gleicher Weise einem weiteren Mutationsschritt unterworfen werden. Dadurch lässt sich die Anzahl der zu untersuchenden Einzelmutanten signifikant verringern.

Die erfindungsgemäßen Ergebnisse liefern wichtige Informationen in bezug auf Struktur und Sequenz der betreffenden Enzyme, die erforderlich sind, um gezielt weitere Enzyme mit gewünschten modifizierten Eigenschaften zu generieren. Insbesondere können sogenannte "hot spots" definiert werden, d.h. Sequenzabschnitte, die sich potentiell eignen, um über die Einführung gezielter Mutationen eine Enzymeigenschaft zu modifizieren.

Nicht limitierende Beispiele solcher hot spot-Regionen der erfindungsgemäßen HSDH sind im Folgenden zusammengefasst:

35-40, insbesondere 37-38, (jeweils bezogen auf die Aminosäuresequenz von HSDH_kurz (SEQ ID NO: 4).
90-105, 93-100 oder 96-100, insbesondere 97 und/oder 98, (jeweils bezogen auf die Aminosäuresequenz von HSDH_kurz (SEQ ID NO: 4)
sowie die korrespondierenden Positionen von SEQ ID NO:2, 6 und 12.

### 3.2.2 Konstrukte

Gegenstand der Erfindung sind außerdem Expressionskonstrukte, enthaltend unter der genetischen Kontrolle regulativer Nukleinsäuresequenzen eine für ein erfindungsgemäßes Polypeptid kodierende Nukleinsäuresequenz; sowie Vektoren, umfassend wenigstens eines dieser Expressionskonstrukte.

Unter einer "Expressionseinheit" wird erfindungsgemäß eine Nukleinsäure mit Expressionsaktivität verstanden, die einen Promotor, wie hierein definiert umfasst, und nach funktioneller Verknüpfung mit einer zu exprimierenden Nukleinsäure oder einem Gen, die Expression, also die Transkription und die Translation dieser Nukleinsäure oder dieses Gens reguliert. Man spricht deshalb auch in diesem Zusammenhang von einer "regulativen Nukleinsäuresequenz". Zusätzlich zum Promotor können weitere, regulative Elemente, wie z.B. Enhancer, enthalten sein.

Unter einer "Expressionskassette" oder "Expressionskonstrukt" wird erfindungsgemäß eine Expressionseinheit verstanden, die mit der zu exprimierenden Nukleinsäure oder dem zu exprimierenden Gen funktionell verknüpft ist. Im Gegensatz zu einer Expressionseinheit umfasst eine Expressionskassette somit nicht nur Nukleinsäuresequenzen, welche Transkription und Translation regulieren, sondern auch die Nukleinsäuresequenzen, welche als Folge der Transkription und Translation als Protein exprimiert werden sollen.

Die Begriffe "Expression" oder "Überexpression" beschreiben im Kontext der Erfindung die Produktion bzw. Erhöhung der intrazellulären Aktivität eines oder mehrerer Enzyme in einem Mikroorganismus, die durch die entsprechende DNA kodiert werden. Dazu kann man beispielsweise ein Gen in einen Organismus einbringen, ein vorhandenes Gen durch ein anderes Gen ersetzen, die Kopienzahl des Gens bzw. der Gene erhöhen, einen starken Promotor verwenden oder ein Gen verwenden, das für ein entsprechendes Enzym mit einer hohen Aktivität kodiert und man kann gegebenenfalls diese Maßnahmen kombinieren.

Vorzugsweise umfassen solche erfindungsgemäßen Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz.

Unter einem "Promotor", einer "Nukleinsäure mit Promotoraktivität" oder einer "Promotorsequenz" wird erfindungsgemäß eine Nukleinsäure verstanden, die in funktioneller Verknüpfung mit einer zu transkribierenden Nukleinsäure die Transkription dieser Nukleinsäure reguliert.

Unter einer "funktionellen" oder "operativen" Verknüpfung versteht man in diesem Zusammenhang beispielsweise die sequentielle Anordnung einer der Nukleinsäuren mit Promotoraktivität und einer zu transkribierenden Nukleinsäuresequenz und gegebenenfalls weiterer regulativer Elemente, wie zum Beispiel Nukleinsäuresequenzen, die die Transkription von Nukleinsäuren gewährleisten, sowie zum Beispiel einen Terminator, derart, dass jedes der regulativen Elemente seine Funktion bei der Transkription der Nukleinsäuresequenz erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben. Bevorzugt sind Anordnungen, in denen die zu transkribierende Nukleinsäuresequenz hinter (d.h. am 3'-Ende) der Promotorsequenz positioniert wird, so dass beide Sequenzen kovalent miteinander verbunden sind. Dabei kann der Abstand zwischen der Promotorsequenz und der transgen zu exprimierende Nukleinsäuresequenz geringer als 200 Basenpaare, oder kleiner als 100 Basenpaare oder kleiner als 50 Basenpaare sein.

Neben Promotoren und Terminator sind als Beispiele weiterer regulativer Elemente zu nennen Targeting-Sequenzen, Enhancer, Polyadenylierungssignale, selektierbare Marker, Amplifikationssignale, Replikationsursprünge und dergleichen. Geeignete regulatorische Sequenzen sind z.B. beschrieben in Goeddel, Gene Expression Technology: Methods in Enzymology 185, Academic Press, San Diego, CA (1990).

Erfindungsgemäße Nukleinsäurekonstrukte umfassen insbesondere Sequenz SEQ ID NO: 1 oder 3 oder Derivate und Homologe davon, sowie die davon ableitbaren Nukleinsäuresequenzen, die mit einem oder mehreren Regulationssignalen vorteilhafterweise zur Steuerung, z.B. Erhöhung, der Genexpression operativ oder funktionell verknüpft wurden.

Zusätzlich zu diesen Regulationssequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde. Das Nukleinsäurekonstrukt kann aber auch einfacher aufgebaut sein, das heißt es wurden keine zusätzlichen Regulationssignale vor die kodierende Sequenz insertiert und der natürliche Promotor mit seiner Regulation wurde nicht entfernt. Stattdessen wird die natürliche Regulationssequenz so mutiert, dass keine Regulation mehr erfolgt und die Genexpression gesteigert wird.

Ein bevorzugtes Nukleinsäurekonstrukt enthält vorteilhafterweise auch eine oder mehrere der schon erwähnten

"Enhancer" Sequenzen, funktionell verknüpft mit dem Promotor, die eine erhöhte Expression der Nukleinsäuresequenz ermöglichen. Auch am 3'-Ende der DNA-Sequenzen können zusätzliche vorteilhafte Sequenzen inseriert werden, wie weitere regulatorische Elemente oder Terminatoren. Die erfindungsgemäßen Nukleinsäuren können in einer oder mehreren Kopien im Konstrukt enthalten sein. Im Konstrukt können noch weitere Marker, wie Antibiotikaresistenzen oder Auxotrophien komplementierende Gene, gegebenenfalls zur Selektion auf das Konstrukt enthalten sein.

Beispiele geeigneter Regulationssequenzen sind in Promotoren wie cos-, tac-, trp-, tet-, trp-tet-, Ipp-, lac-, Ipp-lac-, $lacI^{q-}$, T7-, T5-, T3-, gal-, trc-, ara-, rhaP ($rhaP_{BAD}$)SP6-, lambda-$P_R$- oder im lambda-$P_L$-Promotor enthalten, die vorteilhafterweise in gram-negativen Bakterien Anwendung finden. Weitere vorteilhafte Regulationssequenzen sind beispielsweise in den grampositiven Promotoren amy und SP02, in den Hefe- oder Pilzpromotoren ADC1, MFalpha , AC, P-60, CYC1, GAPDH, TEF, rp28, ADH enthalten. Es können auch künstliche Promotoren für die Regulation verwendet werden.

Das Nukleinsäurekonstrukt wird zur Expression in einem Wirtsorganismus vorteilhafterweise in einen Vektor, wie beispielsweise einem Plasmid oder einem Phagen inseriert, der eine optimale Expression der Gene im Wirt ermöglicht. Unter Vektoren sind außer Plasmiden und Phagen auch alle anderen dem Fachmann bekannten Vektoren, also z.B. Viren, wie SV40, CMV, Baculovirus und Adenovirus, Transposons, IS-Elemente, Phasmide, Cosmide, und lineare oder zirkuläre DNA zu verstehen. Diese Vektoren können autonom im Wirtsorganismus repliziert oder chromosomal repliziert werden. Diese Vektoren stellen eine weitere Ausgestaltung der Erfindung dar.

Geeignete Plasmide sind beispielsweise in E. coli pLG338, pACYC184, pBR322, pUC18, pUC19, pKC30, pRep4, pHS1, pKK223-3, pDHE19.2, pHS2, pPLc236, pMBL24, pLG200, pUR290, $pIN-III^{113}$-B1, λgt11 oder pBdCl, in Streptomyces pIJ101, pIJ364, pIJ702 oder pIJ361, in Bacillus pUB110, pC194 oder pBD214, in Corynebacterium pSA77 oder pAJ667, in Pilzen pALS1, pIL2 oder pBB116, in Hefen 2alphaM, pAG-1, YEp6, YEp13 oder pEMBLYe23 oder in Pflanzen pLGV23, $pGHlac^+$, pBIN19, pAK2004 oder pDH51. Die genannten Plasmide stellen eine kleine Auswahl der möglichen Plasmide dar. Weitere Plasmide sind dem Fachmann wohl bekannt und können beispielsweise aus dem Buch Cloning Vectors (Eds. Pouwels P. H. et al. Elsevier, Amsterdam-New York-Oxford, 1985 , ISBN 0 444 904018) entnommen werden.

In einer weiteren Ausgestaltungsform des Vektors kann der das erfindungsgemäße Nukleinsäurekonstrukt oder die erfindungsgemäße Nukleinsäure enthaltende Vektor auch vorteilhafterweise in Form einer linearen DNA in die Mikroorganismen eingeführt werden und über heterologe oder homologe Rekombination in das Genom des Wirtsorganismus integriert werden. Diese lineare DNA kann aus einem linearisierten Vektor wie einem Plasmid oder nur aus dem Nukleinsäurekonstrukt oder der erfindungsgemäßen Nukleinsäure bestehen.

Für eine optimale Expression heterologer Gene in Organismen ist es vorteilhaft die Nukleinsäuresequenzen entsprechend des im Organismus verwendeten spezifischen "Codonnutzung" zu verändern. Der "Codonnutzung" lässt sich anhand von Computerauswertungen anderer, bekannter Gene des betreffenden Organismus leicht ermitteln.

Die Herstellung einer erfindungsgemäßen Expressionskassette erfolgt durch Fusion eines geeigneten Promotors mit einer geeigneten kodierenden Nukleotidsequenz sowie einem Terminator- oder Polyadenylierungssignal. Dazu verwendet man gängige Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley Interscience (1987) beschrieben sind.

Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden.

### 3.3 Mikroorganismen

Je nach Zusammenhang kann unter dem Begriff "Mikroorganismus" der Ausgangsmikroorganismus (Wildtyp) oder ein genetisch veränderter, rekombinanter Mikroorganismus oder beides verstanden werden.

Mit Hilfe der erfindungsgemäßen Vektoren sind rekombinante Mikroorganismen herstellbar, welche beispielsweise mit wenigstens einem erfindungsgemäßen Vektor transformiert sind und zur Produktion der erfindungsgemäßen Polypeptide eingesetzt werden können. Vorteilhafterweise werden die oben beschriebenen erfindungsgemäßen rekombinanten Konstrukte in ein geeignetes Wirtssystem eingebracht und exprimiert. Dabei werden vorzugsweise dem Fachmann bekannte geläufige Klonierungs- und Transfektionsmethoden, wie beispielsweise Co-Präzipitation, Protoplastenfusion, Elektroporation, retrovirale Transfektion und dergleichen, verwendet, um die genannten Nukleinsäuren im jeweiligen Expressionssystem zur Expression zu bringen. Geeignete Systeme werden beispielsweise in Current Protocols in Molecular Biology, F. Ausubel et al., Hrsg., Wiley Interscience, New York 1997, oder Sambrook et al. Molecular Cloning: A Laboratory Manual. 2. Aufl., Cold Spring Harbor Laboratory, Cold Spring Harbor Laboratory Press, Cold

Spring Harbor, NY, 1989 beschrieben.

Als rekombinante Wirtsorganismen für die erfindungsgemäße Nukleinsäure oder dem Nukleinsäurekonstrukt kommen prinzipiell alle prokaryontischen oder eukaryontischen Organismen in Frage. Vorteilhafterweise werden als Wirtsorganismen Mikroorganismen wie Bakterien, Pilze oder Hefen verwendet. Vorteilhaft werden gram-positive oder gram-negative Bakterien, bevorzugt Bakterien der Familien Enterobacteriaceae, Pseudomonadaceae, Rhizobiaceae, Streptomycetaceae oder Nocardiaceae, besonders bevorzugt Bakterien der Gattungen Escherichia, Pseudomonas, Streptomyces, Nocardia, Burkholderia, Salmonella, Agrobacterium, Clostridium oder Rhodococcus verwendet. Ganz besonders bevorzugt ist die Gattung und Art Escherichia coli. Weitere vorteilhafte Bakterien sind darüber hinaus in der Gruppe der alpha-Proteobacterien, beta-Proteobacterien oder gamma-Proteobacterien zu finden

Der Wirtsorganismus oder die Wirtsorganismen gemäß der Erfindung enthalten dabei vorzugsweise mindestens eine der in dieser Erfindung beschriebenen Nukleinsäuresequenzen, Nukleinsäurekonstrukte oder Vektoren, die für ein Enzym mit 12α-HSDH-Aktivität gemäß obiger Definition kodieren.

Die im erfindungsgemäßen Verfahren verwendeten Organismen werden je nach Wirtsorganismus in dem Fachmann bekannter Weise angezogen bzw. gezüchtet. Mikroorganismen werden in der Regel in einem flüssigen Medium, das eine Kohlenstoffquelle meist in Form von Zuckern, eine Stickstoffquelle meist in Form von organischen Stickstoffquellen wie Hefeextrakt oder Salzen wie Ammoniumsulfat, Spurenelemente wie Eisen-, Mangan-, Magnesiumsalze und gegebenenfalls Vitamine enthält, bei Temperaturen zwischen 0 °C und 100 °C, bevorzugt zwischen 10 °C bis 60 °C unter Sauerstoffbegasung angezogen. Dabei kann der pH der Nährflüssigkeit auf einen festen Wert gehalten werden, das heißt während der Anzucht reguliert werden oder nicht. Die Anzucht kann "batch"-weise, "semi batch"-weise oder kontinuierlich erfolgen. Nährstoffe können zu Beginn der Fermentation vorgelegt oder semikontinuierlich oder kontinuierlich nachgefüttert werden.

### 3.4 Herstellung von UDCS

Zur medikamentösen Behandlung von Gallensteinleiden werden seit vielen Jahren u. a. die Wirkstoffe Ursodesoxycholsäure (UDCS) und das zugehörige Diastereomer Chenodesoxycholsäure (CDCS) eingesetzt. Beide Verbindungen unterscheiden sich lediglich durch die Konfiguration der Hydroxygruppe am C-Atom 7 (UDCS: β -Konfiguration, CDCS: α-Konfiguration). Zur Herstellung von kommerziellen Mengen UDCS wird bisher bevorzugt ein Verfahren verwendet, bei dem CDCS als Rohstoff eingesetzt wird. CDCS wiederum wird vorzugsweise aus Cholsäure (CS) hergestellt.

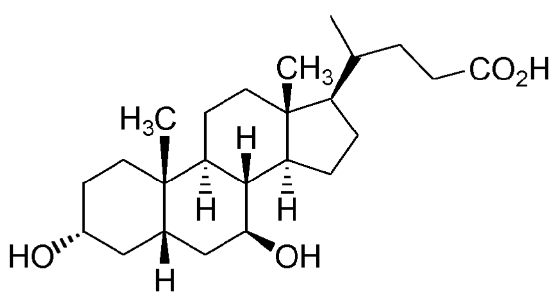

UDCS

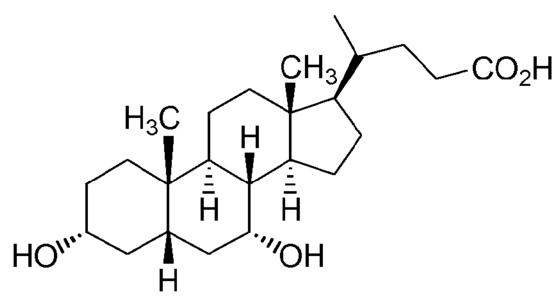

CDCS

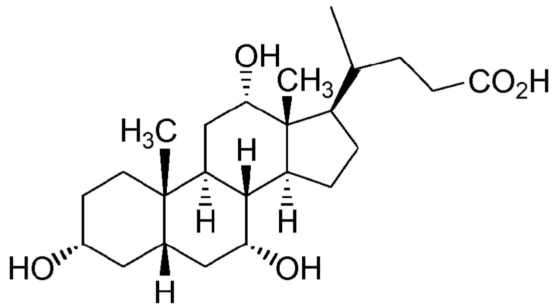

CS

Als Rohstoff für die Herstellung von CDCS (CAS 474-25-9) wird CS (CAS 81-25-4) verwendet.

Als Alternative zum ausschließlich chemischen Verfahren wird erfindungsgemäß eine enzymkatalysierte Oxidation von CS zu 12-Ketochenodesoxycholsäure (12-Keto-CDCS, CAS 2458-08-4), die dann weiter zu CDCS umgesetzt wird, bereitgestellt. Dieser Syntheseweg beinhaltet nur zwei Schritte und ist damit im Vergleich zu der rein chemischen Route deutlich einfacher durchzuführen.

*1. Schritt: enzymatische Oxidation*

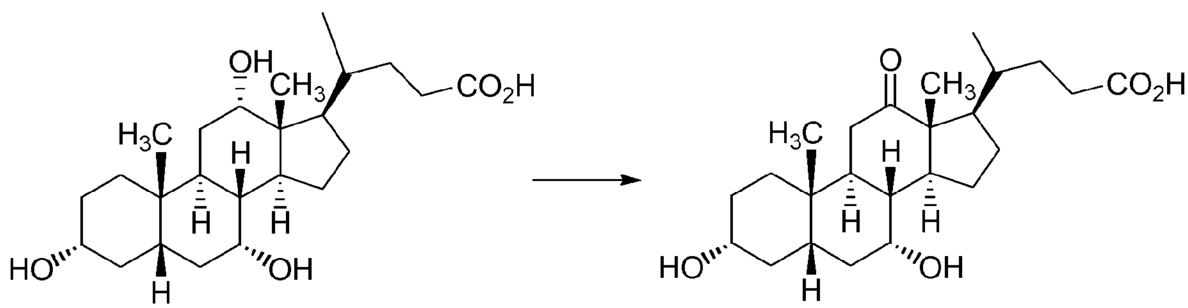

CS 12-Keto-CDCS

*2. Schritt: Desoxygenierung*

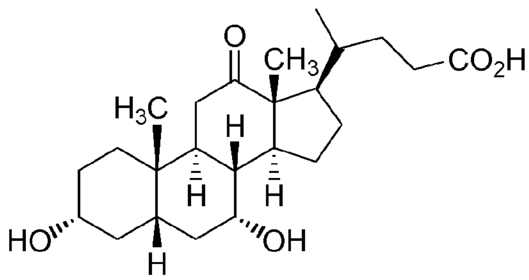

12-Keto-CDCS

CDCS

Gemäß Schritt 1 wird Cholsäure mittels einer 12α-HSDH-Mutante zu 12-Ketochenodesoxycholsäure (12-Keto-CDCS) $NADP^+$-abhängig oxidiert. Diese Reaktion ist reversibel.

Die enzymatische Oxidation erfolgt erfindungsgemäß bevorzugt mittels einer erfindungsgemäßen 12α-HSDH-Mutante und Kofaktorregenerierung. Zur Cofaktorregenerierung können dazu an sich bekannte Systeme gemäß Stand der Technik verwendet werden. So lehrt der Stand der Technik beispielsweise die Verwendung von Glutamat Dehydrogenase (GLDH) welche in freier oder immobilisierter Form (vgl. Carrea, G. et al. Biotechnology and Bioengineering, 1984, 26(5):p. 560-563) verwendet werden kann. Die GLDH reoxidiert NADPH zu $NADP^+$ unter gleichzeitiger reduktiver Aminierung von α-Ketoglutarat zu Glutamat. Alternative dazu werden auch Alkoholdehydrogenasen, wie ADH-Tb, ADH-Lb und ADH-Ms, zur Cofaktorregenerierung vorgeschlagen (Fossati, E. et al., Biotechnol Bioeng., 2006, 93(6):p. 1216-20). Die ADH wandelt Aceton in 2-Propanol unter Regenerierung von $NADP^+$ um. Einen Überblick über obige und weitere potentiell geeignete Enzyme zur Cofaktorregenerierung findet man in Kroutil, W. et al., Adv. Synth. Catal. 2004, 346, 125-142, worazf hiermit ausdrücklich Bezug genommen wird. Dort werden auch NADH-Oxidasen zur Regeneration von $NAD(P)^+$ vorgeschlagen, wobei Sauerstoff zu Wasser reduziert und NAD(P)H zu $NAD(P)^+$ oxidiert wird; ferner werden Flavin Mononukleotid (FMN) Reduktasen vorgeschlagen, welche in gegenwart von Sauerstof NAD(P)H zu $NAD(P)^+$ unter Bildung von wasserstoffperoxid oxidieren. Ebenfalls erwüähnt sind elektrochemische und photochemische verfahren zur $NADP^+$ Regenerierung.

Die Desoxygenierung gemäß Schritt 2 ist eine klassische chemische Wolff-Kishner-Reduktion. Ein wesentlicher Vorteil dieser Route ist, dass durch die Selektivität des Enzyms die Verunreinigung Lithocholsäure nicht entsteht.

Als Rohstoff für die Herstellung von UDCS (CAS 128-13-2) wird CDCS verwendet. Im ersten Syntheseschritt wird die Hydroxylgruppe in Position 7 der CDCS zum entsprechenden Keton oxidiert. Es resultiert die 7-Ketolithocholsäure (3α-Hydroxy-7-ketocholansäure, kurz: KLCS, CAS 4651-67-6). Im zweiten Schritt folgt die stereoselektive Reduktion der Ketogruppe in Position 7. Ziel ist es, mit möglichst hoher Diastereoselektivität UDCS zu erhalten. In der Regel enthält die UDCS direkt nach der Reduktion noch einige Prozent des Diastereomers CDCS. Um zum Wirkstoff UDCS zu gelangen, muss UDCS roh in einem dritten Schritt gereinigt werden.

*1. Schritt: Oxidation*

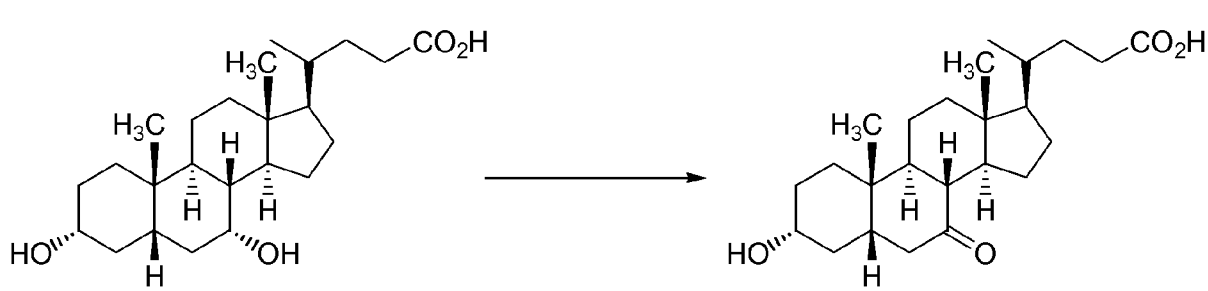

CDCS

KLCS

*2. Schritt: Reduktion*

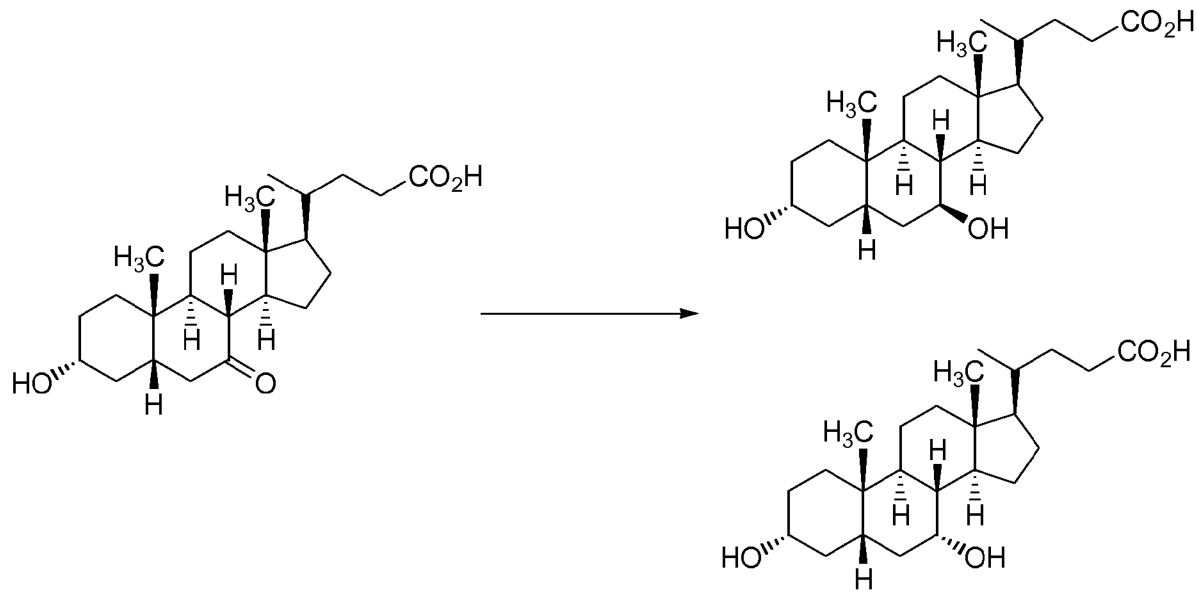

KLCS

UDCS roh (bestehend aus UDCS und CDCS)

*3. Schritt: Reinigung*

UDCS roh -> UDCS rein

Die Oxidation der CDCS erfolgt üblicherweise mit wässriger Natriumhypochloritlösung. In der Literatur findet sich noch die Chromsäureoxidation als Alternative. KLCS fällt als Feststoff an, der dann im zweiten Schritt weiter verarbeitet wird. Die Reduktion kann mit Natriummetall in Alkoholen durchgeführt werden. Es resultiert ein Rohprodukt mit einer Zusammensetzung von UDCS : CDCS von ca. 85 : 15. In alternativen Verfahren wird KLCS mit Wasserstoff an einem Nickelkatalysator (Raney-Nickel) in Alkoholen (wie z.B. aliphatischen Alkoholen) als Lösungsmittel zusammen mit einer Base, wie Kalium-t-butylat oder Kaliumhydroxid, reduziert (EP-A-0 230 085). Zusätzlich ist noch die Reduktion mit Kalium und Lithium (höhere Selektivität als Natrium, C. Giordano et al. Angew. Chem. 1985, 97, 510) sowie Zink (ES 489661) und elektrochemisch (US 4 547 271) möglich.

Bei der Aufreinigung von UDCS roh zu UDCS rein handelt es sich um eine Trennung diastereomerer Salze. Sie erfolgt durch Herstellung, Isolierung und nachfolgende Spaltung eines geeigneten Salzes von UDCS. In der Literatur werden folgende alternativen Reinigungsmethoden genannt: Herstellung, Umkristallisation und Spaltung eines korrespondierenden UDCS-Esters (EP-A-0386 538), Extraktionen (JP 60006699) und chromatographische Verfahren.(IT 2000MI1177).

**3.5 Rekombinante Herstellung von 12α-HSDH-Mutanten**

Gegenstand der Erfindung sind weiterhin Verfahren zur rekombinanten Herstellung erfindungsgemäße Polypeptide oder funktioneller, biologisch aktiver Fragmente davon, wobei man einen Polypeptide-produzierenden Mikroorganismus kultiviert, gegebenenfalls die Expression der Polypeptide induziert und diese aus der Kultur isoliert. Die Polypeptide können so auch in großtechnischem Maßstab produziert werden, falls dies erwünscht ist.

Die erfindungsgemäß hergestellten Mikroorganismen können kontinuierlich oder diskontinuierlich im batch-Verfahren (Satzkultivierung) oder im fed batch (Zulaufverfahren) oder repeated fed batch Verfahren (repetitives Zulaufverfahren) kultiviert werden. Eine Zusammenfassung über bekannte Kultivierungsmethoden ist im Lehrbuch von Chmiel (Bioprozeßtechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) zu finden.

Das zu verwendende Kulturmedium hat in geeigneter Weise den Ansprüchen der jeweiligen Stämme zu genügen. Beschreibungen von Kulturmedien verschiedener Mikroorganismen sind im Handbuch "Manual of Methods für General Bacteriology" der American Society für Bacteriology (Washington D. C., USA, 1981) enthalten.

Diese erfindungsgemäß einsetzbaren Medien umfassen gewöhnlich eine oder mehrere Kohlenstoffquellen, Stickstoffquellen, anorganische Salze, Vitamine und/oder Spurenelemente.

Bevorzugte Kohlenstoffquellen sind Zucker, wie Mono-, Di- oder Polysaccharide. Sehr gute Kohlenstoffquellen sind beispielsweise Glucose, Fructose, Mannose, Galactose, Ribose, Sorbose, Ribulose, Lactose, Maltose, Saccharose, Raffinose, Stärke oder Cellulose. Man kann Zucker auch über komplexe Verbindungen, wie Melassen, oder andere Nebenprodukte derZucker-Raffinierung zu den Medien geben. Es kann auch vorteilhaft sein, Gemische verschiedener Kohlenstoffquellen zuzugeben. Andere mögliche Kohlenstoffquellen sind Öle und Fette wie z. B. Sojaöl. Sonnenblumenöl. Erdnußöl und Kokosfett, Fettsäuren wie z. B. Palmitinsäure, Stearinsäure oder Linolsäure, Alkohole wie z. B. Glycerin, Methanol oder Ethanol und organische Säuren wie z. B. Essigsäure oder Milchsäure.

Stickstoffquellen sind gewöhnlich organische oder anorganische Stickstoffverbindungen oder Materialien, die diese Verbindungen enthalten. Beispielhafte Stickstoffquellen umfassen Ammoniak-Gas oder Ammoniumsalze, wie Ammoniumsulfat, Ammoniumchlorid, Ammoniumphosphat, Ammoniumcarbonat oder Ammoniumnitrat, Nitrate, Harnstoff, Aminosäuren oder komplexe Stickstoffquellen, wie Maisquellwasser, Sojamehl, Sojaprotein, Hefeextrakt, Fleischextrakt und andere. Die Stickstoffquellen können einzeln oder als Mischung verwendet werden.

Anorganische Salzverbindungen, die in den Medien enthalten sein können, umfassen die Chlorid-, Phosphor- oder Sulfatsalze von Calcium, Magnesium, Natrium, Kobalt, Molybdän, Kalium, Mangan, Zink, Kupfer und Eisen.

Als Schwefelquelle können anorganische schwefelhaltige Verbindungen wie beispielsweise Sulfate, Sulfite, Dithionite, Tetrathionate, Thiosulfate, Sulfide aber auch organische Schwefelverbindungen, wie Mercaptane und Thiole, verwendet werden.

Als Phosphorquelle können Phosphorsäure, Kaliumdihydrogenphosphat oder Dikaliumhydrogenphosphat oder die entsprechenden Natrium-haltigen Salze verwendet werden.

Chelatbildner können zum Medium gegeben werden, um die Metallionen in Lösung zu halten. Besonders geeignete Chelatbildner umfassen Dihydroxyphenole, wie Catechol oder Protocatechuat, oder organische Säuren, wie Citronensäure.

Die erfindungsgemäß eingesetzten Fermentationsmedien enthalten üblicherweise auch andere Wachstumsfaktoren, wie Vitamine oder Wachstumsförderer, zu denen beispielsweise Biotin, Riboflavin, Thiamin, Folsäure, Nikotinsäure, Panthothenat und Pyridoxin gehören. Wachstumsfaktoren und Salze stammen häufig von komplexen Medienkomponenten, wie Hefeextrakt, Melassen, Maisquellwasser und dergleichen. Dem Kulturmedium können überdies geeignete Vorstufen zugesetzt werden. Die genaue Zusammensetzung der Medienverbindungen hängt stark vom jeweiligen Experiment ab und wird für jeden spezifischen Fall individuell entschieden. Information über die Medienoptimierung ist erhältlich aus dem Lehrbuch "Applied Microbiol. Physiology, A Practical Approach" (Hrsg. P.M. Rhodes, P.F. Stanbury, IRL Press (1997) S. 53-73, ISBN 0 19 963577 3). Wachstumsmedien lassen sich auch von kommerziellen Anbietern beziehen, wie Standard 1 (Merck) oder BHI (Brain heart infusion, DIFCO) und dergleichen.

Sämtliche Medienkomponenten werden, entweder durch Hitze (20 min bei 1,5 bar und 121°C) oder durch Sterilfiltration, sterilisiert. Die Komponenten können entweder zusammen oder nötigenfalls getrennt sterilisiert werden. Sämtliche Medienkomponenten können zu Beginn der Anzucht zugegen sein oder wahlfrei kontinuierlich oder chargenweise hinzugegeben werden.

Die Temperatur der Kultur liegt normalerweise zwischen 15°C und 45°C, vorzugsweise bei 25°C bis 40°C und kann während des Experimentes konstant gehalten oder verändert werden. Der pH-Wert des Mediums sollte im Bereich von 5 bis 8,5, vorzugsweise um 7,0 liegen. Der pH-Wert für die Anzucht lässt sich während der Anzucht durch Zugabe von basische Verbindungen wie Natriumhydroxid, Kaliumhydroxid, Ammoniak bzw. Ammoniakwasserodersaure Verbindungen wie Phosphorsäure oder Schwefelsäure kontrollieren. Zur Kontrolle der Schaumentwicklung können Antischaummittel, wie z. B. Fettsäurepolyglykolester, eingesetzt werden. Zur Aufrechterhaltung der Stabilität von Plasmiden können dem Medium geeignete selektiv wirkende Stoffe, wie z. B. Antibiotika, hinzugefügt werden. Um aerobe Bedingungen aufrechtzuerhalten, werden Sauerstoff oder Sauerstoff-haltige Gasmischungen, wie z. B. Umgebungsluft, in die Kultur eingetragen. Die Temperatur der Kultur liegt normalerweise bei 20°C bis 45°C und. Die Kultur wird so lange fortgesetzt, bis sich ein Maximum des gewünschten Produktes gebildet hat. Dieses Ziel wird normalerweise innerhalb von 10 Stunden bis 160 Stunden erreicht.

Die Fermentationsbrühe wird anschließend weiterverarbeitet. Je nach Anforderung kann die Biomasse ganz oder teilweise durch Separationsmethoden, wie z. B. Zentrifugation, Filtration, Dekantieren oder einer Kombination dieser Methoden aus der Fermentationsbrühe entfernt oder vollständig in ihr belassen werden.

Die Zellen können auch, falls die Polypeptide nicht in das Kulturmedium sezerniert werden, aufgeschlossen und das Produkt nach bekannten Proteinisolierungsverfahren aus dem Lysat gewonnen. Die Zellen können wahlweise durch hochfrequenten Ultraschall, durch hohen Druck, wie z.B. in einer French-Druckzelle, durch Osmolyse, durch Einwirkung von Detergenzien, lytischen Enzymen oder organischen Lösungsmitteln, durch Homogenisatoren oder durch Kombination mehrerer der aufgeführten Verfahren aufgeschlossen werden.

Eine Aufreinigung der Polypeptide kann mit bekannten, chromatographischen Verfahren erzielt werden, wie Molekularsieb-Chromatographie (Gelfiltration), wie Q-Sepharose-Chromatographie, Ionenaustausch-Chromatographie und hydrophobe Chromatographie, sowie mit anderen üblichen Verfahren wie Ultrafiltration, Kristallisation, Aussalzen, Dialyse und nativer Gelelektrophorese. Geeignete Verfahren werden beispielsweise in Cooper, F. G., Biochemische Arbeitsmethoden, Verlag Walter de Gruyter, Berlin, New York oder in Scopes, R., Protein Purification, Springer Verlag, New York, Heidelberg, Berlin beschrieben.

Vorteilhaft kann es sein, zur Isolierung des rekombinanten Proteins Vektorsysteme oder Oligonukleotide zu verwenden, die die cDNA um bestimmte Nukleotidsequenzen verlängern und damit für veränderte Polypeptide oder Fusionsproteine kodieren, die z.B. einer einfacheren Reinigung dienen. Derartige geeignete Modifikationen sind beispielsweise als Anker fungierende sogenannte "Tags", wie z.B. die als Hexa-Histidin-Anker bekannte Modifikation oder Epitope, die als Antigene von Antikörpern erkannt werden können (beschrieben zum Beispiel in Harlow, E. and Lane, D., 1988, Antibodies: A Laboratory Manual. Cold Spring Harbor (N.Y.) Press). Diese Anker können zur Anheftung der Proteine an einen festen Träger, wie z.B. einer Polymermatrix, dienen, die beispielsweise in einer Chromatographiesäule eingefüllt sein kann, oder an einer Mikrotiterplatte oder an einem sonstigen Träger verwendet werden kann.

Gleichzeitig können diese Anker auch zur Erkennung der Proteine verwendet werden. Zur Erkennung der Proteine können außerdem übliche Marker, wie Fluoreszenzfarbstoffe, Enzymmarker, die nach Reaktion mit einem Substrat ein detektierbares Reaktionsprodukt bilden, oder radioaktive Marker, allein oder in Kombination mit den Ankern zur Derivatisierung der Proteine verwendet werden.

### 3.6 Enzymimmobilisierung

Die erfindungsgemäßen Enzyme können in den hierin beschriebenen Verfahren frei oder immobilisiert eingesetzt werden. Unter einem immobilisierten Enzym versteht man ein Enzym, das an einen inerten Träger fixiert ist. Geeignete Trägermaterialien sowie die darauf immobilisierten Enzyme sind aus der EP-A-1149849, EP-A-1 069 183 und der DE-OS 100193773 sowie aus den darin zitierten Literaturstellen bekannt. Auf die Offenbarung dieser Schriften wird diesbezüglich in vollem Umfang Bezug genommen. Zu den geeigneten Trägermaterialien gehören beispielsweise Tone, Tonmineralien, wie Kaolinit, Diatomeenerde, Perlit, Siliciumdioxid, Aluminiumoxid, Natriumcarbonat, Calciumcarbonat, Cellulosepulver, Anionenaustauschermaterialien, synthetische Polymere, wie Polystyrol, Acrylharze, Phenolformaldehydharze, Polyurethane und Polyolefine, wie Polyethylen und Polypropylen. Die Trägermaterialien werden zur Herstellung der geträgerten Enzyme üblicherweise in einer feinteiligen, partikelförmigen Form eingesetzt, wobei poröse Formen bevorzugt sind. Die Partikelgröße des Trägermaterials beträgt üblicherweise nicht mehr als 5 mm, insbesondere nicht mehr als 2 mm (Sieblinie). Analog kann bei Einsatz der Dehydrogenase als Ganzzell-Katalysator eine freie oder immobiliserte Form gewählt werden. Trägermaterialien sind z.B. Ca-Alginat, und Carrageenan. Enzyme wie auch Zellen können auch direkt mit Glutaraldehyd vernetzt werden (Cross-linking zu CLEAs). Entsprechende und weitere Immobilisierungsverfahren sind beispielsweise in J. Lalonde und A. Margolin "Immobilization of Enzymes" "in K. Drauz und H. Waldmann, Enzyme Catalysis in Organic Synthesis 2002, Vol.III, 991-1032, Wiley-VCH, Weinheim beschrieben.

## EXPERIMENTELLER TEIL

Werden keine anderen Angaben gemacht, so können die im Rahmen der vorliegenden Erfindung durchgeführten Klonierungsschritte, wie z.B. Restriktionsspaltungen, Agarose Gelelektrophorese, Reinigung von DNA-Fragmenten, Transfer von Nukleinsäuren auf Nitrozellulose und Nylonmembranen, Verknüpfen von DNA-Fragmenten, Transformation von Mikroorganismen, Anzucht von Mikroorganismen, Vermehrung von Phagen und Sequenzanalyse rekombinanter DNA wurden wie bei Sambrook et al. (1989) a.a.O. beschrieben durchgeführt werden.

### A. Allgemeine Angaben

**Materialien:**

Enzyme und Enzympuffer wurden von Fermentas, St. Leon-Rot oder NEB, Frankfurt bezogen.

| LB-Medium: | |
|---|---|
| Bacto-Trypton | 10 g |
| Hefe-Extrakt | 5 g |
| Natriumchlorid | 5 g |
| doppeltdestilliertes Wasser | ad 1000 ml |

| TB-Medium: | |
|---|---|
| Lösung I: | |
| Bacto-Trypton | 12 g |
| Hefe-Extrakt | 24 |
| Glycerin, wasserfrei | 4 ml |
| doppeltdestilliertes Wasser | ad 900 ml |
| | |
| Lösung II: | |
| Kaliumdihydrogenphosphat | 0,17 M |
| Kaliumhydrogenphosphat | 0,72 M |
| doppeltdestilliertes Wasser | ad 100 ml |

Beide Lösungen wurden nach dem Autoklavieren vereinigt.

## Expressionsvektoren

Zur Expression von 12α-HSDH diente der Vektor pET22b(+), Novagen, Darmstadt, welcher eine MCS unter der Kontrolle eines T7-Promotors und -Transkriptionsstarts enthält und einen T7-Terminator besitzt. Die Expression wird mittels Isopropyl-β-D-thiogalactopyranosid (IPTG) induziert.

Dazu wurden 12α-HSDH kodierende Sequenzen PCR-amplifiziert. Die PCR-Produkte erhielt man unter Verwendung der genomischen DNA von *Clostridium sp.* group P strain 48-50 als Template und der später noch genauer beschriebenen Primerpaare. Die PCR-Produkte wurden auf ein Agarose-Gel aufgetragen, aufgetrennt und aus diesem ausgeschnitten. Anschließend wurden sie mit Hilfe von Ndel und BamHI restringiert und mit dem ebenfalls mit Ndel und BamHI geschnittenen pET22b(+)-Vektor ligiert.

## Mikroorganismen

| Stamm | Genotyp |
|---|---|
| *Clostridium sp.* group P strain 48-50 | |
| *Escherichia coli* BL21 (DE3) | $F^-$ ompT gal dcm Ion $hsdS_B(r_B^- m_B^-)$ λ(DE3 [lacl lacUV5-T7 gene 1 ind1 sam7 nin5]) |

## Methoden

### 1. Standardbedingungen für 12α-HSDH Aktivitätsbestimmung

Die Aktivität wird wie folgt definiert: 1 U des Enzyms entspricht der Enzymmenge, welche die Umsetzung von 1 μmol/min einer 5 mM Cholsäurelösung in Kaliumphosphat-Puffer (50 mM, pH 8,0) bei Raumtemperatur (d.h. ca. 20 °C-23 °C) katalysiert.

Zur Aktivitätsbestimmung wurden 790 μl Kaliumphosphatpuffer (50 mM, pH 8,0), 100 μl Cholsäure (50 mM in Kaliumphosphatpuffer (50 mM, pH 8,0)) und 10 μl zu vermessende Enzymlösung in einer Küvette gemischt. Zum Start der Reaktion wurde 100 μl $NADP^+$ (2,5 mM) zugegeben und die Zunahme der Absorption bei 340 nm photometrisch bestimmt. Die Steigung über 30 s wurde bei RT ermittelt. Die Bestimmung der Aktivität erfolgte nach dem Lambert-Beerschen-Gesetz.

## 2. Proteinkonzentrationsbestimmung mittels BCA-Assay

Die Proteinkonzentration einer Lösung wurde bestimmt, indem die Absorption von 20 μl Proteinlösung, wie z.B. einer Zelllysat-Verdünnung bzw. eines resuspendierten Zelldebris-Pellets nach Ultraschallaufschluss, in 200 μl BCA-Lösung (Lösung A:Lösung B 50:1) des Analysen-Kits der Firma Bio-Rad, München bei 562 nm gemessen wurde. Dabei bildet der Bicinchoninsäure (BCA) mit einwertigen Kupferionen, die quantitativ aus der Reduktion zweiwertigen Kupferionen durch das Protein entstehen, eine violette Komplexverbindung, dessen Absorption bei 562 nm photometrisch gemessen werden kann. Die Bestimmung der Konzentration erfolgte über eine Rinderserumalbumin (BSA)-Eichgerade.

## B. Herstellung und Untersuchung von 12α-HSDH-Mutanten

### Beispiel 1: Herstellung der 12α-HSDH-Mutanten G38A und G38S

Die 12α-HSDH kodierende Sequenz wurde wie in der WO 2009/118176 erstmals beschrieben exprimiert und isoliert.

Es wurde die Position 38 der Aminosäuresequenz (vgl. SEQ I D NO:6) mutiert (vgl. auch Figur 1).

### 1.1. Primer

*Glycin → Alanin*

Forward: MBr_QC_HSDH_G37A_forw:

TGGTCCTGACCGCCAGAAACGAGCA (SEQ ID NO:7)

Revers: MBr_QC_HSDH_G37A_rev:

TGCTCGTTTCTGGCGGTCAGGACCA (SEQ ID NO:8)

*Glycin → Serin*

Forward: MBr_QC_HSDH_G37S_forw:

TGGTCCTGACCAGCAGAAACGAGCAGAAA (SEQ ID NO: 9)

Revers: MBr_QC_HSDH_G37S_rev:

TTTCTGCTCGTTTCTGCTGGTCAGGACCA (SEQ ID NO:10)

### 1.2. PCR-Programm

Bei der Reaktion erfolgte zuerst ein 5 min langer initialer Denaturierungsschritt bei 95°C. Danach wurden 18 Zyklen von Denaturierung (45 s bei 95°C), Primerhybridisierung (1,5 min bei 54°C) und Elongation (12,5 min bei 72°C) durchgeführt. Als letzter Schritt erfolgte eine finale Elongation von 15 min bei 72°C bevor die Polymerasekettenreaktion durch Kühlen auf 4°C beendet wurde.

### 1.3. PCR-Ansatz

| | |
|---|---|
| Pfu Puffer (10x) | 5 μl |
| dNTP-Mix (20 mM) | 1 μl |
| *Forward*-Primer (10 μM) | 2 μl |
| *Revers*-Primer (10 μM) | 2 μl |
| Templat | 1 μl |

(fortgesetzt)

| | |
|---|---|
| Pfu Polymerase | 1 μl |
| DMSO | 2,5 μl |
| $ddH_2O$ | 35,5 μl |
| | **50 μl** |

Als Templat wurde ein pET22b-Vektor mit der 12α-HSDH verwendet (wie im entsprechenden Patent).

**1.4. Durchführung**

Um Aminosäuren in Proteinsequenzen gezielt austauschen zu können wird die DNA-Sequenz des entsprechenden Gens positionsgerichtet mutiert. Hierzu werden zueinander komplementäre Primer genutzt, die die gewünschte Mutation in ihrer Sequenz tragen. Als Templat dient N6-adeninmethylierte, doppelsträngige Plasmid-DNA, die das zu mutierende Gen tragen. N6-adeninmethylierte Plasmid-DNA werden aus $dam^+$ *E. coli*-Stamm wie zum Beispiel *E. coli* DH5 isoliert.

Die Polymerasekettenreaktion wird wie oben beschrieben durchgeführt. Dabei werden die Primer komplementär zum Templat verlängert, wodurch Plasmide mit der gewünschten Mutation entstehen, die einen Strangbruch aufweisen. Anders als bei anderen PCR-Reaktionen steigt die DNA-Ausbeute hierbei nur linear, da neu gebildete DNA-Moleküle nicht als Templat für die PCR-Reaktion dienen können.

Nach Abschluss der PCR-Reaktion wird die parentale, N6-adeninmethylierte mit Hilfe des Restriktionsenzyms DpnI verdaut. Dieses Enzym hat die Besonderheit, dass es unspezifisch N6-adeninmethylierte DNA restringiert, jedoch nicht die neu gebildete, nichtmethylierte DNA. Die Restriktion erfolgte, indem 1 μL DpnI zum PCR-Reaktionsansatz hinzu gegeben und 1 h bei 37°C inkubiert wurde.

10 μl dieses Ansatzes wurden zur Transformation von 200 μl chemisch-kompetenten DH5α-Zellen verwendet. Nach Plasmidisolation wurde die erfolgreiche Mutation mittels Sequenzierung bestätigt.

**<u>Beispiel 2:</u> Bestimmung der Umsatzraten der Mutanten G38S und G38A**

Für jede der Mutanten sowie für das Wildtyp-Enzym wurden Kinetikmessungen im Mikrotiterplattenphotometer durchgeführt, indem die Umsatzraten der Enzyme sowohl mit gleichbleibenden Substratkonzentrationen bei variierten Kofaktorkonzentrationen als auch *vice versa* aufgezeichnet wurden. Zur Bestimmung der spezifischen Enzymaktivität wurden die Enzymkonzentrationen im Zelllysat densitometrisch bestimmt.

Zur Ermittlung der Abhängigkeit der Substratumsatzgeschwindigkeit von der Substratkonzentration wurde eine Kofaktorkonzentration von 100 μM $NADP^+$ eingesetzt, während die Substratkonzentration über einen Bereich von 6 μM bis 10 mM Cholsäure mit 32 verschiedenen Konzentrationen variiert wurde.

Zur Ermittlung der Abhängigkeit der Substratumsatzgeschwindigkeit von der Kofaktorkonzentration wurde eine Substratkonzentration von 10 mM Cholsäure bezogen auf das Reaktionsvolumen eingesetzt, während die Kofaktorkonzentration über einen Bereich von 2 μM bis 100 μM $NADP^+$ mit 16 verschiedenen Konzentrationen beim Wildtypprotein bzw. über einen Bereich von 2 μM bis 1000 μM $NADP^+$ mit 24 verschiedenen Konzentrationen bei den beiden Mutanten variiert wurde (jeweils bezogen auf den Raktionsansatz).

Über das eingesetzte Volumen an Zelllysat lässt sich die volumenbezogene Enzymaktivität berechnen. Zusammen mit den bestimmten Enzymkonzentrationen lassen sich die spezifischen Enzymaktivitäten berechnen.

Die erhaltenen Daten wurden mit IGOR Pro ausgewertet. Dabei wurden für jedes Protein die Werte für jeweils eingesetzte Substratkonzentration bzw. Kofaktorkonzentration gegen die spezifische Enzymaktivität aufgetragen. Aus den Auftragungen der Substrat- bzw. Kofaktorkonzentration gegen die spezifische Enzymaktivität lässt sich für alle Reaktionen der typische Kurvenverlauf einer Michaelis-Menten-Kinetik erkennen. Aus dem Grund wurde das Michaelis-Menten-Modell für die Auswertung verwendet. Die Werte für $v_{max}$, $K_m$ wurden durch nichtlineare Regression ermittelt.

Mit den aufgezeigten Daten und der Datenauswertung lassen sich Halbsättigungskonzentrationen für Cholsäure $K_{m,CS}$ und $NADP^+$ $K_{m,\ NADP}$ sowie maximale spezifische Enzymaktivitäten bei unendlicher Cholsäure-Konzentration ($v_{max,CS}$) bwz. bei unendlicher $NADP^+$-Konzentration ($v_{max,\ NADP}$) bestimmen. Die absolute maximale Enzymaktivität bei unendlicher Cholsäure-Konzentration und gleichzeitig unendlicher $NADP^+$-Konzentration ($v_{max}$) erhält man nach der Formel:

$$v_{max} = v_{max,CS} \cdot \frac{K_{m,NADP} + c_{NADP}}{c_{NADP}}$$

Die ermittelten Parameter für die drei verschiedenen 12α-HSDH-Varianten sind in folgender Tabelle 1 aufgeführt und in Figur 2 grafisch dargestellt.

**Tabelle 1: Enzymkinetische Parameter der 12α-HSDH (WT) und der Mutanten G38A und G38S mit verbesserter Aktivität**

| | $K_{m,CS}$, μM | $K_{m,NADP}$, μM | $V_{max}$, U $mg^{-1}$ |
|---|---|---|---|
| WT | 136 ± 12 | 21,4 ± 2,3 | 40,1 ± 2,8 |
| G38A | 299 ± 26 | 159 ± 9 | 168 ± 12 |
| G38S | 252 ± 27 | 334 ± 10 | 287 ± 13 |

Durch die Mutation der Position G38 konnte die spezifische Aktivität $v_{max}$ überraschenderweise gegenüber dem Wildtyp unter Verwendung des Cofaktors $NADP^+$ um das vier- bzw. sieben-fache erhöht werden (Tabelle 1). Es ist davon auszugehen, dass unter Verwendung der gezeigten, neuartigen Mutanten die notwendige Enzymkonzentration verringert werden kann, wodurch sich Kosten und Produktaufarbeitungsaufwand erheblich reduzieren lassen.

Zuordnung der SEQ ID NOs:

| SEQ ID NO: | Beschreibung | Typ |
|---|---|---|
| 1 | 12α-HSDH; L | NS |
| 2 | 12α-HSDH; L | AS |
| 3 | 12α-HSDH; K | NS |
| 4 | 12α-HSDH; K | AS |
| 5 | 12α-HSDH; K; + Met | NS |
| 6 | 12α-HSDH; K; + Met | AS |
| 7 | PCR Primer | NS |
| 8 | PCR Primer | NS |
| 9 | PCR Primer | NS |
| 10 | PCR Primer | NS |
| 11 | Mutante 37D12; K | NS |
| 12 | Mutante 37D12 ; K | AS |
| AS = Aminosäuresequenz<br>NS= Nukleinsäuresequenz<br>L= Langversion<br>K= Kurzversion | | |

Auf die Offenbarung der hierin zitierten Publikationen wird ausdrücklich Bezug genommen.

**Patentansprüche**

1. 12α-Hydroxysteroiddehydrogenase (12α-HSDH)-Mutante, welche wenigstens die stereospezifische enzymatische Oxidation eines 12-Hydroxysteroids zum korrespondierenden 12-Ketosteroid katalysiert, wobei die Mutante im Vergleich zum nicht-mutierten Enzym eine erhöhte spezifische Aktivität (U/mg) in Gegenwart des Cofaktors $NADP^+$ aufweist.

2. Mutante nach Anspruch 1, wobei spezifische Aktivität (U/mg) in Gegenwart des Cofaktors $NADP^+$ um wenigsten 1, 5 oder 10% erhöht ist, insbesondere aber wenigstens um das 1-fache, insbesondere um das 2- bis 10-fache erhöht ist.

3. 12α-Hydroxysteroiddehydrogenase (12α -HSDH)-Mutante, welche wenigstens die stereospezifische enzymatische Oxidation eines 12-Hydroxysteroids zum korrespondierenden 12-Ketosteroid katalysiert, wobei die Mutante wenigstens eine Mutation in der Aminosäure-Sequenz gemäß SEQ ID NO: 2, 4, 6 oder 12 oder einer davon abgeleiteten Aminosäuresequenz mit wenigstens 60% Sequenzidentität aufweist.

4. Mutante nach einem der vorhergehenden Ansprüche, wobei die Mutante wenigstens eine Mutation im Sequenzmotiv VLTGRNE gemäß Position 35 bis 41 von SEQ ID NO:6 oder gemäß Position 34 bis 40 von SEQ ID NO:4; oder gemäß Position 47 bis 53 von SEQ ID NO:2; oder gemäß Position 35 bis 41 von SEQ ID NO:12 oder in dem korrespondierende Sequenzmotiv einer davon abgeleiteten Aminosäuresequenz mit wenigstens 60% Sequenzidentität aufweist.

5. Mutante nach Anspruch 4, ausgewählt unter den Einfachmutanten G→X, worin X für irgendeine natürliche, von G verschiedene Aminosäure steht, und insbesondere ausgewählt unter dem Mutanten G→A, G→S, G→V, G→P, G→N, G→E, G→Q, G→H, G→R, G→K und G→W oder den korrespondierenden Einfachmutanten einer davon abgeleiteten Aminosäuresequenz mit wenigstens 60% Sequenzidentität.

6. Mutante nach einem der Ansprüche 3 bis 5, wobei die Mutation ausgewählt ist unter G38A und G38S nach SEQ ID NO: 6.

7. Nukleinsäuresequenz kodierend für eine 12α-HSDH Mutante nach einem der vorhergehenden Ansprüche.

8. Expressionskassette, umfassend eine Nukleinsäuresequenz gemäß Anspruch 7 unter der genetischen Kontrolle wenigstens einer regulativen Nukleinsäuresequenz.

9. Vektor, umfassend wenigstens eine Expressionskassette nach Anspruch 8.

10. Rekombinanter Mikroorganismus, der wenigstens eine Nukleinsäuresequenz gemäß Anspruch 7 oder wenigstens eine Expressionskassette nach Anspruch 8 trägt oder mit wenigstens einem Vektor nach Anspruch 9 transformiert ist.

11. Verfahren zur enzymatischen Synthese von 12α-Hydroxysteroiden, wobei man das korrespondierende 12-Ketosteroid in Gegenwart einer 12α -HSDH Mutante gemäß der Definition in einem der Ansprüche 1 bis 6 umsetzt, und wenigstens ein gebildetes Reduktionsprodukt aus dem Reaktionsansatz gegebenenfalls isoliert.

12. Verfahren nach Anspruch 11, wobei die Reduktion in Gegenwart (und unter Verbrauch) von NADPH erfolgt.

13. Verfahren zur enzymatischen Oxidation von 12α-Hydroxysteroiden, wobei man das Hydroxysteroid in Gegenwart einer 12α -Hydroxysteroiddehydrogenase gemäß der Definition in einem der Ansprüche 1 bis 6 umsetzt, und ein gebildetes Oxidationsprodukt aus dem Reaktionsansatz gegebenenfalls isoliert.

14. Verfahren nach Anspruch 13, wobei die Oxidation in Gegenwart (und unter Verbrauch) von $NADP^+$ erfolgt.

15. Verfahren nach Anspruch 14, wobei verbrauchtes $NADP^+$ durch Kopplung mit einem $NADP^+$-regenerierenden Enzym ausgewählt unter einer $NADP^+$-Dehydrogenase und insbesondere einer Alkoholdehydrogenase (ADH), wie ausgewählt unter natürlichen oder rekombinanten, isolierten oder angereicherten

   a) Alkoholdehydrogenasen (EC 1.1.1.2) und
   b) davon abgeleiteten funktionalen Äquivalenten,

regeneriert wird.

**16.** Verfahren zur Herstellung von Ursodesoxycholsäure (UDCS) der Formel (1)

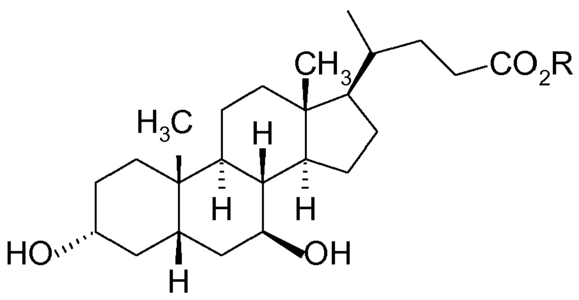

(1)

worin

R für Alkyl, $NR^1R^2$, H, ein Alkalimetallion oder $N(R^3)_4^+$steht, worin die Reste $R^3$ gleich oder verschieden sind und für H oder Alkyl stehen,

wobei man

a) eine Cholsäure (CS) der Formel (2)

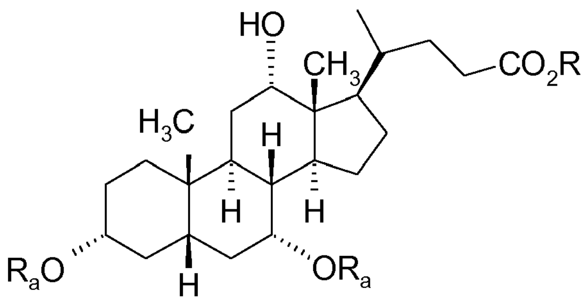

(2)

worin R die oben angegebenen Bedeutungen besitzen, und die Reste $R_a$ gleich oder verschieden sind und für H oder Acyl stehen, in Gegenwart einer 12α-Hydroxysteroiddehydrogenase-Mutante nach einem der Ansprüche 1 bis 6 zur korrespondierenden 12-Ketochenodesoxycholsäure (12-Keto CDCS) der Formel (3)

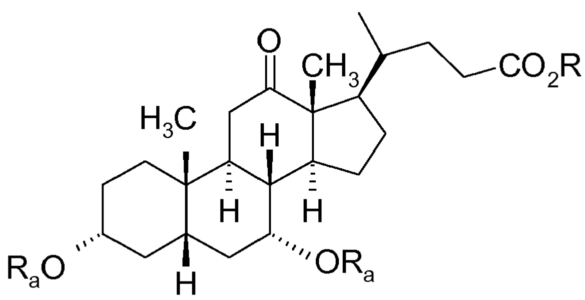

(3)

worin R und $R_a$ die oben angebebenen Bedeutungen besitzen, oxidiert und anschließend
b) 12-Keto-CDCS der Formel (3) durch Desoxygenierung zu Chenodesoxycholsäure (CDCS) der Formel (4)

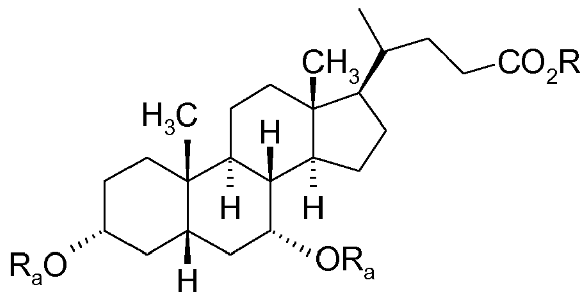

(4)

worin R und $R_a$ die oben angebebenen Bedeutungen besitzen, umsetzt und

c) CDCS der Formel (4) in Position 7 chemisch oxidiert zur 7-Keto-Lithocholsäure (KLCS) der Formel (5)

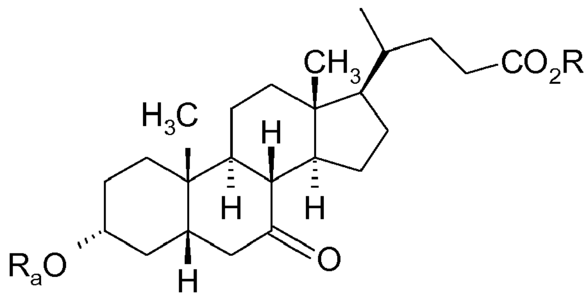

(5)

worin R und $R_a$ die oben angebebenen Bedeutungen besitzen; und

d) KLCS der Formel (5) reduziert und

e) das Reaktionsprodukt gegebenenfalls weiter aufreinigt.

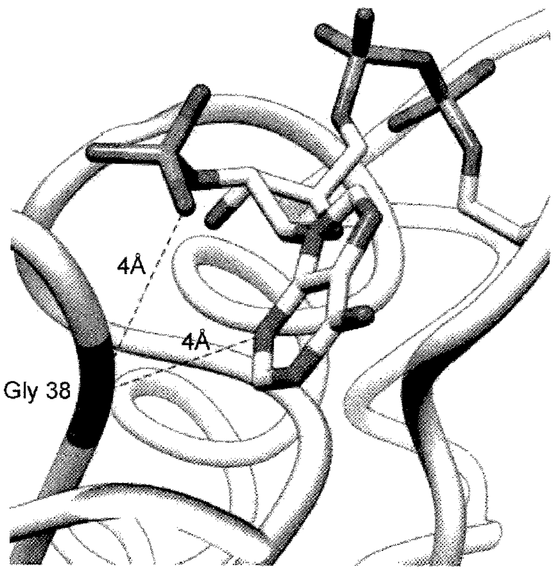


Fig. 1

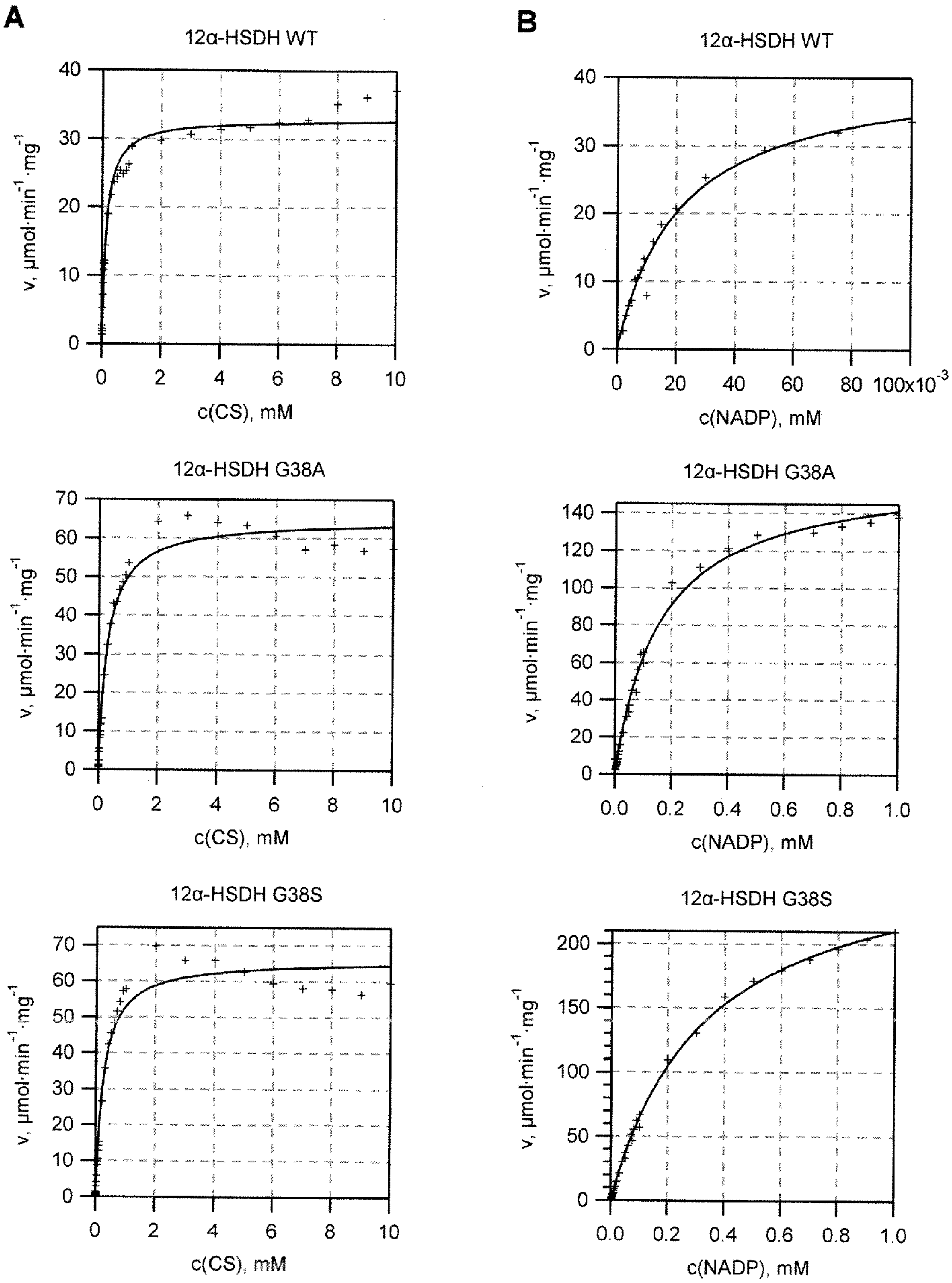
A
B
12α-HSDH WT
12α-HSDH G38A
12α-HSDH G38S
v, µmol·min$^{-1}$·mg$^{-1}$
c(CS), mM
c(NADP), mM
100x10$^{-3}$

Fig. 2

Europäisches Patentamt
European Patent Office
Office européen des brevets

# EUROPÄISCHER RECHERCHENBERICHT

Nummer der Anmeldung
EP 10 18 7411

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 2 105 500 A1 (PHARMAZELL GMBH [DE]) 30. September 2009 (2009-09-30) * Ansprüche 8-33; Beispiel 5 * ----- | 1-5,7-16 | INV. C07K14/33 C12N9/04 |

RECHERCHIERTE SACHGEBIETE (IPC)
C07K
C12N

Der vorliegende Recherchenbericht wurde für alle Patentansprüche erstellt

1

| Recherchenort | Abschlußdatum der Recherche | Prüfer |
|---|---|---|
| München | 27. Mai 2011 | Stoyanov, Borislav |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 10 18 7411

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

27-05-2011

| Im Recherchenbericht angeführtes Patentdokument | Datum der Veröffentlichung | Mitglied(er) der Patentfamilie | Datum der Veröffentlichung |
|---|---|---|---|
| EP 2105500 A1 | 30-09-2009 | AU 2009230728 A1 | 01-10-2009 |
| | | CN 102007210 A | 06-04-2011 |
| | | EP 2268803 A2 | 05-01-2011 |
| | | WO 2009118176 A2 | 01-10-2009 |
| | | KR 20110014980 A | 14-02-2011 |
| | | US 2011091921 A1 | 21-04-2011 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1731618 A
- WO 2009118176 A
- WO 0188172 A
- EP 0230085 A
- ES 489661
- US 4547271 A
- EP 0386538 A
- JP 60006699 B
- IT 2000MI1177
- EP 1149849 A
- EP 1069183 A
- DE OS100193773 A


### In der Beschreibung aufgeführte Nicht-Patentliteratur


- **HARRIS ; HYLEMON.** *Biochim Biophys Acta,* 1978, vol. 528 (1), 148-57
- **MACDONALD et al.** *Biochim Biophys Acta,* 1976, vol. 450 (2), 142-53
- **MAHONY, D.E. et al.** *Appl Environ Microbiol,* 1977, vol. 34 (4), 419-23
- **MACDONALD, I.A. et al.** *Journal of Lipid Research,* 1979, vol. 20, 234-239
- **SUTHERLAND, J.D. et al.** *Prep Biochem,* 1982, vol. 12 (4), 307-21
- **BRAUN, M. et al.** *Eur J Biochem,* 1991, vol. 196 (2), 439-50
- **R.K. SCOPES.** Protein Purification. Springer Verlag, 1982
- **PEARSON ; LIPMAN.** *Proc. Natl. Acad, Sci. (USA,* 1988, vol. 85 (8), 2444-2448
- **NARANG, S.A.** *Tetrahedron,* 1983, vol. 39, 3
- **ITAKURA et al.** *Annu. Rev. Biochem.,* 1984, vol. 53, 323
- **ITAKURA et al.** *Science,* 1984, vol. 198, 1056
- **IKE et al.** *Nucleic Acids Res.,* 1983, vol. 11, 477
- **ARKIN ; YOURVAN.** *PNAS,* 1992, vol. 89, 7811-7815
- **DELGRAVE et al.** *Protein Engineering,* 1993, vol. 6 (3), 327-331
- **HIGGINS DG ; SHARP PM.** Fast and sensitive multiple sequence alignments on a microcomputer. *Comput Appl. Biosci.,* April 1989, vol. 5 (2), 151-1
- **CHENNA ; RAMU ; SUGAWARA ; HIDEAKI ; KO-IKE ; TADASHI ; LOPEZ ; RODRIGO ; GIBSON ; TOBYJ.** Multiple sequence alignment with the Clustal series of programs. *Nucleic Acids Res,* 2003, vol. 31 (13), 3497-500
- Voet, Voet. Wiley Press, 896-897
- **SAMBROOK et al.** Molecular Cloning: A laboratory manual. Cold Spring Harbor Laboratory Press, 1989
- **SAMBROOK, J. ; FRITSCH, E.F. ; MANIATIS, T.** Molecular Cloning: A Laboratory Manual. 2. Aufl., Cold Spring Harbor Laboratory. Cold Spring Harbor Laboratory Press, 1989
- **SAMBROOK et al.** Molecular Cloning. Cold Spring Harbor Laboratory, 1989
- Current Protocols in Molecular Biology. John Wiley & Sons, 1985
- Nucleic Acids Hybridization: A Practical Approach. IRL Press at Oxford University Press, 1985
- Essential Molecular Biology: A Practical Approach. IRL Press at Oxford University Press, 1991
- **SAMBROOK, J. ; FRITSCH, E.F. ; MANIATIS, T.** Molecular Cloning (A Laboratory Manual. Cold Spring Harbor Laboratory Press, 1989, 9.31-9.57
- Current Protocols in Molecular Biology. John Wiley & Sons, 1989, 6.3.1-6.3.6
- **SAMBROOK ; RUSSELL.** Molecular Cloning. Cold Spring Harbor Laboratory Press, 2001
- In vitro mutagenesis protocols. Humana Press, 1996
- **KEGLER-EBO DM ; DOCKTOR CM ; DIMAIO D.** *Nucleic Acids Res,* 1994, vol. 22, 1593
- **BARETTINO D ; FEIGENBUTZ M ; VALCÄREL R ; STUNNENBERG HG.** *Nucleic Acids Res,* 1994, vol. 22, 541
- **BARIK S.** *Mol Biotechnol,* 1995, vol. 3, 1
- **ECKERT KA ; KUNKEL TA.** *Nucleic Acids Res,* 1990, vol. 18, 3739
- An efficient random mutagenesis technique using an E.coli mutator strain. **GREENER A ; CALLAHAN M ; JERPSETH B.** In vitro mutagenesis protocols. Humana Press, 1996
- **REETZ MT ; JAEGER K-E.** *Topics Curr Chem,* 1999, vol. 200, 31

- Methods for optimizing industrial enzymes by directed evolution. **ZHAO H ; MOORE JC ; VOLKOV AA ; ARNOLD FH.** Manual of industrial microbiologyand biotechnology. American Society for Microbiology, 1999
- **GOEDDEL.** Gene Expression Technology: Methods in Enzymology. Academic Press, 1990, vol. 185
- Buch Cloning Vectors. Elsevier, 1985
- **T. MANIATIS ; E.F. FRITSCH ; J. SAMBROOK.** Molecular Cloning: A Laboratory Manual. Cold Spring Harbor Laboratory, 1989
- **T.J. SILHAVY ; M.L. BERMAN ; L.W. ENQUIST.** Experiments with Gene Fusions. Cold Spring Harbor Laboratory, 1984
- **AUSUBEL, F.M. et al.** Current Protocols in Molecular Biology. Greene Publishing Assoc. and Wiley Interscience, 1987
- Cloning Vectors. Elsevier, 1985
- Current Protocols in Molecular Biology. Wiley Interscience, 1997
- **SAMBROOK et al.** Molecular Cloning: A Laboratory Manual. Cold Spring Harbor Laboratory Press, 1989
- **CARREA, G. et al.** *Biotechnology and Bioengineering,* 1984, vol. 26 (5), 560-563
- **FOSSATI, E. et al.** *Biotechnol Bioeng.,* 2006, vol. 93 (6), 1216-20
- **KROUTIL, W. et al.** *Adv. Synth. Catal.,* 2004, vol. 346, 125-142
- **C. GIORDANO et al.** *Angew. Chem.,* 1985, vol. 97, 510
- Bioverfahrenstechnik. Gustav Fischer Verlag, 1991
- **STORHAS.** Bioreaktoren und periphere Einrichtungen. Vieweg Verlag, 1994
- **HANDBUCH.** Manual of Methods für General Bacteriology. American Society für Bacteriology, 1981
- Applied Microbiol. Physiology, A Practical Approach. IRL Press, 1997, 53-73
- **COOPER, F. G.** Biochemische Arbeitsmethoden. Verlag Walter de Gruyter
- **SCOPES, R.** Protein Purification. Springer Verlag
- **HARLOW, E. ; LANE, D.** Antibodies: A Laboratory Manual. Cold Spring Harbor (N.Y.) Press, 1988
- Immobilization of Enzymes. **J. LALONDE ; A. MARGOLIN.** Enzyme Catalysis in Organic Synthesis. Wiley-VCH, 2002, vol. III, 991-1032

SEQUENCE LISTING

<110> PHARMAZELL GmbH

<120> 12alpha-HSDH-Mutanten

<130> M/51186

<160> 12

<170> PatentIn version 3.3

<210> 1
<211> 813
<212> DNA
<213> Clostridium sp.

<220>
<221> CDS
<222> (1)..(813)

<400> 1
atg gat ttt att gat ttt aag gag atg ggc aga atg ggg atc ttt gac 48
Met Asp Phe Ile Asp Phe Lys Glu Met Gly Arg Met Gly Ile Phe Asp
1 5 10 15

gga aag gtc gca atc att act ggc ggg ggc aag gcc aaa tcg atc ggc 96
Gly Lys Val Ala Ile Ile Thr Gly Gly Gly Lys Ala Lys Ser Ile Gly
20 25 30

tac ggc att gcc gtg gcc tat gct aag gag ggg gcc aac ctg gtc ctg 144
Tyr Gly Ile Ala Val Ala Tyr Ala Lys Glu Gly Ala Asn Leu Val Leu
35 40 45

acc ggc aga aac gag cag aaa ctg ctg gac gcc aag gag gag ctg gag 192
Thr Gly Arg Asn Glu Gln Lys Leu Leu Asp Ala Lys Glu Glu Leu Glu
50 55 60

cgc ctc tac ggc atc aag gtg ttg ccg ctg gcg gtg gac gtc acc ccc 240
Arg Leu Tyr Gly Ile Lys Val Leu Pro Leu Ala Val Asp Val Thr Pro
65 70 75 80

agc gat gag tcg gag gac cgg gtc aag gaa gcc gtg cag aag gtc atc 288
Ser Asp Glu Ser Glu Asp Arg Val Lys Glu Ala Val Gln Lys Val Ile
85 90 95

gcc gaa ttc ggc cgc atc gac gtg ctg atc aac aac gcc cag gcg tcg 336
Ala Glu Phe Gly Arg Ile Asp Val Leu Ile Asn Asn Ala Gln Ala Ser
100 105 110

gcc tcg ggc atc ccc ctg tcc atg cag acc aaa gac cac ttt gac ctg 384
Ala Ser Gly Ile Pro Leu Ser Met Gln Thr Lys Asp His Phe Asp Leu
115 120 125

ggc atc tac tcc ggg ctc tac gcc acc ttc tac tac atg agg gag tgc 432
Gly Ile Tyr Ser Gly Leu Tyr Ala Thr Phe Tyr Tyr Met Arg Glu Cys
130 135 140

tat ccc tac ctg aag gag acc cag ggc tcg gtc atc aac ttc gcc tcc 480
Tyr Pro Tyr Leu Lys Glu Thr Gln Gly Ser Val Ile Asn Phe Ala Ser
145 150 155 160

ggc gcc ggc ctc ttc ggc aac gtg ggt cag tgc tcc tac gcc gcc gcc 528
Gly Ala Gly Leu Phe Gly Asn Val Gly Gln Cys Ser Tyr Ala Ala Ala
165 170 175

aaa gag ggc atc cgc ggc ctc tcc cgc gtc gcg gcc acc gag tgg ggc 576
Lys Glu Gly Ile Arg Gly Leu Ser Arg Val Ala Ala Thr Glu Trp Gly

```
            180                 185                 190

aag gac aac atc aac gtc aac gtg gtc tgc ccc ctg gcc atg acc gcc        624
Lys Asp Asn Ile Asn Val Asn Val Val Cys Pro Leu Ala Met Thr Ala
        195                 200                 205

cag ctg gag aac ttc aag ctc tcc tac cct gag gcc tac gag aaa aac        672
Gln Leu Glu Asn Phe Lys Leu Ser Tyr Pro Glu Ala Tyr Glu Lys Asn
    210                 215                 220

ctc aga ggg gtg ccc atg ggc cgc ttc ggt gac ccc gag ctg gac atc        720
Leu Arg Gly Val Pro Met Gly Arg Phe Gly Asp Pro Glu Leu Asp Ile
225                 230                 235                     240

ggc cgg gtc tgc gtg cag ctc ggc tcg ccc gac ttc aag tac atg tcc        768
Gly Arg Val Cys Val Gln Leu Gly Ser Pro Asp Phe Lys Tyr Met Ser
                245                 250                 255

ggc gag acc ctc acc ctg gaa ggc ggc atg ggt cag cgc ccc tag            813
Gly Glu Thr Leu Thr Leu Glu Gly Gly Met Gly Gln Arg Pro
            260                 265                 270


<210>  2
<211>  270
<212>  PRT
<213>  Clostridium sp.

<400>  2

Met Asp Phe Ile Asp Phe Lys Glu Met Gly Arg Met Gly Ile Phe Asp
1               5                   10                  15


Gly Lys Val Ala Ile Ile Thr Gly Gly Gly Lys Ala Lys Ser Ile Gly
            20                  25                  30


Tyr Gly Ile Ala Val Ala Tyr Ala Lys Glu Gly Ala Asn Leu Val Leu
        35                  40                  45


Thr Gly Arg Asn Glu Gln Lys Leu Leu Asp Ala Lys Glu Glu Leu Glu
    50                  55                  60


Arg Leu Tyr Gly Ile Lys Val Leu Pro Leu Ala Val Asp Val Thr Pro
65                  70                  75                  80


Ser Asp Glu Ser Glu Asp Arg Val Lys Glu Ala Val Gln Lys Val Ile
                85                  90                  95


Ala Glu Phe Gly Arg Ile Asp Val Leu Ile Asn Asn Ala Gln Ala Ser
            100                 105                 110


Ala Ser Gly Ile Pro Leu Ser Met Gln Thr Lys Asp His Phe Asp Leu
        115                 120                 125


Gly Ile Tyr Ser Gly Leu Tyr Ala Thr Phe Tyr Tyr Met Arg Glu Cys
    130                 135                 140


Tyr Pro Tyr Leu Lys Glu Thr Gln Gly Ser Val Ile Asn Phe Ala Ser
145                 150                 155                 160
```

```
Gly Ala Gly Leu Phe Gly Asn Val Gly Gln Cys Ser Tyr Ala Ala Ala
                165                 170                 175

Lys Glu Gly Ile Arg Gly Leu Ser Arg Val Ala Ala Thr Glu Trp Gly
            180                 185                 190

Lys Asp Asn Ile Asn Val Asn Val Val Cys Pro Leu Ala Met Thr Ala
        195                 200                 205

Gln Leu Glu Asn Phe Lys Leu Ser Tyr Pro Glu Ala Tyr Glu Lys Asn
    210                 215                 220

Leu Arg Gly Val Pro Met Gly Arg Phe Gly Asp Pro Glu Leu Asp Ile
225                 230                 235                 240

Gly Arg Val Cys Val Gln Leu Gly Ser Pro Asp Phe Lys Tyr Met Ser
                245                 250                 255

Gly Glu Thr Leu Thr Leu Glu Gly Gly Met Gly Gln Arg Pro
            260                 265                 270


<210>  3
<211>  774
<212>  DNA
<213>  Clostridium sp.


<220>
<221>  CDS
<222>  (1)..(774)

<400>  3
atc ttt gac gga aag gtc gca atc att act ggc ggg ggc aag gcc aaa       48
Ile Phe Asp Gly Lys Val Ala Ile Ile Thr Gly Gly Gly Lys Ala Lys
1               5                   10                  15

tcg atc ggc tac ggc att gcc gtg gcc tat gct aag gag ggg gcc aac       96
Ser Ile Gly Tyr Gly Ile Ala Val Ala Tyr Ala Lys Glu Gly Ala Asn
            20                  25                  30

ctg gtc ctg acc ggc aga aac gag cag aaa ctg ctg gac gcc aag gag      144
Leu Val Leu Thr Gly Arg Asn Glu Gln Lys Leu Leu Asp Ala Lys Glu
        35                  40                  45

gag ctg gag cgc ctc tac ggc atc aag gtg ttg ccg ctg gcg gtg gac      192
Glu Leu Glu Arg Leu Tyr Gly Ile Lys Val Leu Pro Leu Ala Val Asp
    50                  55                  60

gtc acc ccc agc gat gag tcg gag gac cgg gtc aag gaa gcc gtg cag      240
Val Thr Pro Ser Asp Glu Ser Glu Asp Arg Val Lys Glu Ala Val Gln
65                  70                  75                  80

aag gtc atc gcc gaa ttc ggc cgc atc gac gtg ctg atc aac aac gcc      288
Lys Val Ile Ala Glu Phe Gly Arg Ile Asp Val Leu Ile Asn Asn Ala
                85                  90                  95

cag gcg tcg gcc tcg ggc atc ccc ctg tcc atg cag acc aaa gac cac      336
Gln Ala Ser Ala Ser Gly Ile Pro Leu Ser Met Gln Thr Lys Asp His
            100                 105                 110

ttt gac ctg ggc atc tac tcc ggg ctc tac gcc acc ttc tac tac atg      384
```

```
Phe Asp Leu Gly Ile Tyr Ser Gly Leu Tyr Ala Thr Phe Tyr Tyr Met
        115                 120                 125

agg gag tgc tat ccc tac ctg aag gag acc cag ggc tcg gtc atc aac        432
Arg Glu Cys Tyr Pro Tyr Leu Lys Glu Thr Gln Gly Ser Val Ile Asn
    130                 135                 140

ttc gcc tcc ggc gcc ggc ctc ttc ggc aac gtg ggt cag tgc tcc tac        480
Phe Ala Ser Gly Ala Gly Leu Phe Gly Asn Val Gly Gln Cys Ser Tyr
145                 150                 155                 160

gcc gcc gcc aaa gag ggc atc cgc ggc ctc tcc cgc gtc gcg gcc acc        528
Ala Ala Ala Lys Glu Gly Ile Arg Gly Leu Ser Arg Val Ala Ala Thr
                165                 170                 175

gag tgg ggc aag gac aac atc aac gtc aac gtg gtc tgc ccc ctg gcc        576
Glu Trp Gly Lys Asp Asn Ile Asn Val Asn Val Val Cys Pro Leu Ala
            180                 185                 190

atg acc gcc cag ctg gag aac ttc aag ctc tcc tac cct gag gcc tac        624
Met Thr Ala Gln Leu Glu Asn Phe Lys Leu Ser Tyr Pro Glu Ala Tyr
        195                 200                 205

gag aaa aac ctc aga ggg gtg ccc atg ggc cgc ttc ggt gac ccc gag        672
Glu Lys Asn Leu Arg Gly Val Pro Met Gly Arg Phe Gly Asp Pro Glu
    210                 215                 220

ctg gac atc ggc cgg gtc tgc gtg cag ctc ggc tcg ccc gac ttc aag        720
Leu Asp Ile Gly Arg Val Cys Val Gln Leu Gly Ser Pro Asp Phe Lys
225                 230                 235                 240

tac atg tcc ggc gag acc ctc acc ctg gaa ggc ggc atg ggt cag cgc        768
Tyr Met Ser Gly Glu Thr Leu Thr Leu Glu Gly Gly Met Gly Gln Arg
                245                 250                 255

ccc tag                                                                774
Pro


<210>  4
<211>  257
<212>  PRT
<213>  Clostridium sp.

<400>  4

Ile Phe Asp Gly Lys Val Ala Ile Ile Thr Gly Gly Gly Lys Ala Lys
1               5                   10                  15

Ser Ile Gly Tyr Gly Ile Ala Val Ala Tyr Ala Lys Glu Gly Ala Asn
            20                  25                  30

Leu Val Leu Thr Gly Arg Asn Glu Gln Lys Leu Leu Asp Ala Lys Glu
        35                  40                  45

Glu Leu Glu Arg Leu Tyr Gly Ile Lys Val Leu Pro Leu Ala Val Asp
    50                  55                  60

Val Thr Pro Ser Asp Glu Ser Glu Asp Arg Val Lys Glu Ala Val Gln
65                  70                  75                  80

Lys Val Ile Ala Glu Phe Gly Arg Ile Asp Val Leu Ile Asn Asn Ala
                85                  90                  95
```

```
Gln Ala Ser Ala Ser Gly Ile Pro Leu Ser Met Gln Thr Lys Asp His
            100                 105                 110

Phe Asp Leu Gly Ile Tyr Ser Gly Leu Tyr Ala Thr Phe Tyr Tyr Met
        115                 120                 125

Arg Glu Cys Tyr Pro Tyr Leu Lys Glu Thr Gln Gly Ser Val Ile Asn
    130                 135                 140

Phe Ala Ser Gly Ala Gly Leu Phe Gly Asn Val Gly Gln Cys Ser Tyr
145                 150                 155                 160

Ala Ala Ala Lys Glu Gly Ile Arg Gly Leu Ser Arg Val Ala Ala Thr
                165                 170                 175

Glu Trp Gly Lys Asp Asn Ile Asn Val Asn Val Val Cys Pro Leu Ala
            180                 185                 190

Met Thr Ala Gln Leu Glu Asn Phe Lys Leu Ser Tyr Pro Glu Ala Tyr
        195                 200                 205

Glu Lys Asn Leu Arg Gly Val Pro Met Gly Arg Phe Gly Asp Pro Glu
    210                 215                 220

Leu Asp Ile Gly Arg Val Cys Val Gln Leu Gly Ser Pro Asp Phe Lys
225                 230                 235                 240

Tyr Met Ser Gly Glu Thr Leu Thr Leu Glu Gly Gly Met Gly Gln Arg
                245                 250                 255

Pro


<210>  5
<211>  777
<212>  DNA
<213>  Clostridium sp.


<220>
<221>  CDS
<222>  (1)..(777)

<400>  5
atg atc ttt gac gga aag gtc gca atc att act ggc ggg ggc aag gcc        48
Met Ile Phe Asp Gly Lys Val Ala Ile Ile Thr Gly Gly Gly Lys Ala
1               5                   10                  15

aaa tcg atc ggc tac ggc att gcc gtg gcc tat gct aag gag ggg gcc        96
Lys Ser Ile Gly Tyr Gly Ile Ala Val Ala Tyr Ala Lys Glu Gly Ala
            20                  25                  30

aac ctg gtc ctg acc ggc aga aac gag cag aaa ctg ctg gac gcc aag       144
Asn Leu Val Leu Thr Gly Arg Asn Glu Gln Lys Leu Leu Asp Ala Lys
        35                  40                  45
```

```
gag gag ctg gag cgc ctc tac ggc atc aag gtg ttg ccg ctg gcg gtg        192
Glu Glu Leu Glu Arg Leu Tyr Gly Ile Lys Val Leu Pro Leu Ala Val
    50                  55                  60

gac gtc acc ccc agc gat gag tcg gag gac cgg gtc aag gaa gcc gtg        240
Asp Val Thr Pro Ser Asp Glu Ser Glu Asp Arg Val Lys Glu Ala Val
65                  70                  75                  80

cag aag gtc atc gcc gaa ttc ggc cgc atc gac gtg ctg atc aac aac        288
Gln Lys Val Ile Ala Glu Phe Gly Arg Ile Asp Val Leu Ile Asn Asn
                85                  90                  95

gcc cag gcg tcg gcc tcg ggc atc ccc ctg tcc atg cag acc aaa gac        336
Ala Gln Ala Ser Ala Ser Gly Ile Pro Leu Ser Met Gln Thr Lys Asp
            100                 105                 110

cac ttt gac ctg ggc atc tac tcc ggg ctc tac gcc acc ttc tac tac        384
His Phe Asp Leu Gly Ile Tyr Ser Gly Leu Tyr Ala Thr Phe Tyr Tyr
        115                 120                 125

atg agg gag tgc tat ccc tac ctg aag gag acc cag ggc tcg gtc atc        432
Met Arg Glu Cys Tyr Pro Tyr Leu Lys Glu Thr Gln Gly Ser Val Ile
    130                 135                 140

aac ttc gcc tcc ggc gcc ggc ctc ttc ggc aac gtg ggt cag tgc tcc        480
Asn Phe Ala Ser Gly Ala Gly Leu Phe Gly Asn Val Gly Gln Cys Ser
145                 150                 155                 160

tac gcc gcc gcc aaa gag ggc atc cgc ggc ctc tcc cgc gtc gcg gcc        528
Tyr Ala Ala Ala Lys Glu Gly Ile Arg Gly Leu Ser Arg Val Ala Ala
                165                 170                 175

acc gag tgg ggc aag gac aac atc aac gtc aac gtg gtc tgc ccc ctg        576
Thr Glu Trp Gly Lys Asp Asn Ile Asn Val Asn Val Val Cys Pro Leu
            180                 185                 190

gcc atg acc gcc cag ctg gag aac ttc aag ctc tcc tac cct gag gcc        624
Ala Met Thr Ala Gln Leu Glu Asn Phe Lys Leu Ser Tyr Pro Glu Ala
        195                 200                 205

tac gag aaa aac ctc aga ggg gtg ccc atg ggc cgc ttc ggt gac ccc        672
Tyr Glu Lys Asn Leu Arg Gly Val Pro Met Gly Arg Phe Gly Asp Pro
    210                 215                 220

gag ctg gac atc ggc cgg gtc tgc gtg cag ctc ggc tcg ccc gac ttc        720
Glu Leu Asp Ile Gly Arg Val Cys Val Gln Leu Gly Ser Pro Asp Phe
225                 230                 235                 240

aag tac atg tcc ggc gag acc ctc acc ctg gaa ggc ggc atg ggt cag        768
Lys Tyr Met Ser Gly Glu Thr Leu Thr Leu Glu Gly Gly Met Gly Gln
                245                 250                 255

cgc ccc tag                                                            777
Arg Pro


<210>  6
<211>  258
<212>  PRT
<213>  Clostridium sp.

<400>  6

Met Ile Phe Asp Gly Lys Val Ala Ile Ile Thr Gly Gly Gly Lys Ala
1               5                   10                  15

Lys Ser Ile Gly Tyr Gly Ile Ala Val Ala Tyr Ala Lys Glu Gly Ala
```

```
            20                  25                  30

Asn Leu Val Leu Thr Gly Arg Asn Glu Gln Lys Leu Leu Asp Ala Lys
        35                  40                  45

Glu Glu Leu Glu Arg Leu Tyr Gly Ile Lys Val Leu Pro Leu Ala Val
    50                  55                  60

Asp Val Thr Pro Ser Asp Glu Ser Glu Asp Arg Val Lys Glu Ala Val
65                  70                  75                  80

Gln Lys Val Ile Ala Glu Phe Gly Arg Ile Asp Val Leu Ile Asn Asn
                85                  90                  95

Ala Gln Ala Ser Ala Ser Gly Ile Pro Leu Ser Met Gln Thr Lys Asp
            100                 105                 110

His Phe Asp Leu Gly Ile Tyr Ser Gly Leu Tyr Ala Thr Phe Tyr Tyr
        115                 120                 125

Met Arg Glu Cys Tyr Pro Tyr Leu Lys Glu Thr Gln Gly Ser Val Ile
    130                 135                 140

Asn Phe Ala Ser Gly Ala Gly Leu Phe Gly Asn Val Gly Gln Cys Ser
145                 150                 155                 160

Tyr Ala Ala Ala Lys Glu Gly Ile Arg Gly Leu Ser Arg Val Ala Ala
                165                 170                 175

Thr Glu Trp Gly Lys Asp Asn Ile Asn Val Asn Val Val Cys Pro Leu
            180                 185                 190

Ala Met Thr Ala Gln Leu Glu Asn Phe Lys Leu Ser Tyr Pro Glu Ala
        195                 200                 205

Tyr Glu Lys Asn Leu Arg Gly Val Pro Met Gly Arg Phe Gly Asp Pro
    210                 215                 220

Glu Leu Asp Ile Gly Arg Val Cys Val Gln Leu Gly Ser Pro Asp Phe
225                 230                 235                 240

Lys Tyr Met Ser Gly Glu Thr Leu Thr Leu Glu Gly Gly Met Gly Gln
                245                 250                 255

Arg Pro
```

<210> 7
<211> 25
<212> DNA
<213> Artificial

<220>

<223> PCR Primer

<400> 7
tggtcctgac cgccagaaac gagca 25

<210> 8
<211> 25
<212> DNA
<213> Artificial

<220>
<223> PCR Primer

<400> 8
tgctcgtttc tggcggtcag gacca 25

<210> 9
<211> 29
<212> DNA
<213> Artificial

<220>
<223> PCR Primer

<400> 9
tggtcctgac cagcagaaac gagcagaaa 29

<210> 10
<211> 29
<212> DNA
<213> Artificial

<220>
<223> PCR Primer

<400> 10
tttctgctcg tttctgctgg tcaggacca 29

<210> 11
<211> 777
<212> DNA
<213> Artificial

<220>
<223> Mutant Q97H

<220>
<221> CDS
<222> (1)..(777)

<400> 11
```
atg atc ttt gac gga aag gtc gca atc att act ggc ggg ggc aag gcc      48
Met Ile Phe Asp Gly Lys Val Ala Ile Ile Thr Gly Gly Gly Lys Ala
1               5                   10                  15

aaa tcg atc ggc tac ggc att gcc gtg gcc tat gct aag gag ggg gcc      96
Lys Ser Ile Gly Tyr Gly Ile Ala Val Ala Tyr Ala Lys Glu Gly Ala
            20                  25                  30

aac ctg gtc ctg acc ggc aga aac gag cag aaa ctg ctg gac gcc aag     144
Asn Leu Val Leu Thr Gly Arg Asn Glu Gln Lys Leu Leu Asp Ala Lys
        35                  40                  45

gag gag ctg gag cgc ctc tac ggc atc aag gtg ttg ccg ctg gcg gtg     192
```

```
Glu Glu Leu Glu Arg Leu Tyr Gly Ile Lys Val Leu Pro Leu Ala Val
    50                  55                  60

gac gtc acc ccc agc gat gag tcg gag gac cgg gtc aag gaa gcc gtg      240
Asp Val Thr Pro Ser Asp Glu Ser Glu Asp Arg Val Lys Glu Ala Val
65                  70                  75                  80

cag aag gtc atc gcc gaa ttc ggc cgc atc gac gtg ctg atc aac aac      288
Gln Lys Val Ile Ala Glu Phe Gly Arg Ile Asp Val Leu Ile Asn Asn
                85                  90                  95

gcc cat gcg tcg gcc tcg ggc atc ccc ctg tcc atg cag acc aaa gac      336
Ala His Ala Ser Ala Ser Gly Ile Pro Leu Ser Met Gln Thr Lys Asp
            100                 105                 110

cac ttt gac ctg ggc atc tac tcc ggg ctc tac gcc acc ttc tac tac      384
His Phe Asp Leu Gly Ile Tyr Ser Gly Leu Tyr Ala Thr Phe Tyr Tyr
        115                 120                 125

atg agg gag tgc tat ccc tac ctg aag gag act cag ggc tcg gtc atc      432
Met Arg Glu Cys Tyr Pro Tyr Leu Lys Glu Thr Gln Gly Ser Val Ile
    130                 135                 140

aac ttc gcc tcc ggc gcc ggc ctc ttc ggc aac gtg ggt cag tgc tcc      480
Asn Phe Ala Ser Gly Ala Gly Leu Phe Gly Asn Val Gly Gln Cys Ser
145                 150                 155                 160

tac gcc gcc gcc aaa gag ggc atc cgc ggc ctc tcc cgc gtc gcg gcc      528
Tyr Ala Ala Ala Lys Glu Gly Ile Arg Gly Leu Ser Arg Val Ala Ala
                165                 170                 175

acc gag tgg ggc aag gac aac atc aac gtc aac gtg gtc tgc ccc ctg      576
Thr Glu Trp Gly Lys Asp Asn Ile Asn Val Asn Val Val Cys Pro Leu
            180                 185                 190

gcc atg acc gcc cag ctg gag aac ttc aag ctc tcc tac cct gag gcc      624
Ala Met Thr Ala Gln Leu Glu Asn Phe Lys Leu Ser Tyr Pro Glu Ala
        195                 200                 205

tac gag aaa aac ctc aga ggg gtg ccc atg ggc cgc ttc ggt gac ccc      672
Tyr Glu Lys Asn Leu Arg Gly Val Pro Met Gly Arg Phe Gly Asp Pro
    210                 215                 220

gag ctg gac atc ggc cgg gtc tgc gtg cag ctc ggc tcg ccc gac ttc      720
Glu Leu Asp Ile Gly Arg Val Cys Val Gln Leu Gly Ser Pro Asp Phe
225                 230                 235                 240

aag tac atg tcc ggc gag acc ctc acc ctg gaa ggc ggc atg ggt cag      768
Lys Tyr Met Ser Gly Glu Thr Leu Thr Leu Glu Gly Gly Met Gly Gln
                245                 250                 255

cgc ccc tag                                                          777
Arg Pro
```

<210> 12
<211> 258
<212> PRT
<213> Artificial

<220>
<223> Synthetic Construct

<400> 12

```
Met Ile Phe Asp Gly Lys Val Ala Ile Ile Thr Gly Gly Gly Lys Ala
1               5                   10                  15
```

```
Lys Ser Ile Gly Tyr Gly Ile Ala Val Ala Tyr Ala Lys Glu Gly Ala
            20                  25                  30

Asn Leu Val Leu Thr Gly Arg Asn Glu Gln Lys Leu Leu Asp Ala Lys
        35                  40                  45

Glu Glu Leu Glu Arg Leu Tyr Gly Ile Lys Val Leu Pro Leu Ala Val
    50                  55                  60

Asp Val Thr Pro Ser Asp Glu Ser Glu Asp Arg Val Lys Glu Ala Val
65                  70                  75                  80

Gln Lys Val Ile Ala Glu Phe Gly Arg Ile Asp Val Leu Ile Asn Asn
                85                  90                  95

Ala His Ala Ser Ala Ser Gly Ile Pro Leu Ser Met Gln Thr Lys Asp
            100                 105                 110

His Phe Asp Leu Gly Ile Tyr Ser Gly Leu Tyr Ala Thr Phe Tyr Tyr
        115                 120                 125

Met Arg Glu Cys Tyr Pro Tyr Leu Lys Glu Thr Gln Gly Ser Val Ile
    130                 135                 140

Asn Phe Ala Ser Gly Ala Gly Leu Phe Gly Asn Val Gly Gln Cys Ser
145                 150                 155                 160

Tyr Ala Ala Ala Lys Glu Gly Ile Arg Gly Leu Ser Arg Val Ala Ala
                165                 170                 175

Thr Glu Trp Gly Lys Asp Asn Ile Asn Val Asn Val Val Cys Pro Leu
            180                 185                 190

Ala Met Thr Ala Gln Leu Glu Asn Phe Lys Leu Ser Tyr Pro Glu Ala
        195                 200                 205

Tyr Glu Lys Asn Leu Arg Gly Val Pro Met Gly Arg Phe Gly Asp Pro
    210                 215                 220

Glu Leu Asp Ile Gly Arg Val Cys Val Gln Leu Gly Ser Pro Asp Phe
225                 230                 235                 240

Lys Tyr Met Ser Gly Glu Thr Leu Thr Leu Glu Gly Gly Met Gly Gln
                245                 250                 255

Arg Pro
```